# EUROPEAN PATENT SPECIFICATION

(11) **EP 1 828 165 B1**
(45) Date of publication and mention of the grant of the patent: **20.03.2013**
(21) Application number: 05799006.1
(22) Date of filing: 25.10.2005
(51) Int. Cl.: C07D 401/12, C07D 403/12, C07D 417/12, A61K 31/282, A61K 31/337, A61K 31/407, A61K 31/4745, A61K 31/475, A61K 31/496, A61K 31/506, A61K 31/513, A61K 31/519, A61K 31/52, A61K 31/675, A61K 31/704, A61K 31/7048, A61K 31/7068, A61K 31/7076, A61K 33/24, A61K 38/21, A61K 45/06

(54) **NOVEL AMINOPYRIDINE DERIVATIVES HAVING AURORA A SELECTIVE INHIBITORY ACTION**
NEUE AMINOPYRIDINDERIVATE MIT EINER SELEKTIVAURORA-A-HEMMENDEN WIRKUNG
NOUVEAUX DERIVES D'AMINOPYRIDINE PRESENTANT UNE ACTION D'INHIBITION SELECTIVE DE LA PROTEINE KINASE AURORA A

(30) Priority: 29.10.2004 JP 2004315152; 01.06.2005 JP 2005161156
(43) Date of publication of application: 05.09.2007
(73) Proprietor: MSD K.K., Chiyoda-ku Tokyo 102-8667 (JP)
(72) Inventor: OHKUBO, Mitsuru, BANYU PHARMACEUTICAL CO., LTD., Tsukuba-shi, Ibaraki 3002611 (JP); KATO, Tetsuya, BANYU PHARMACEUTICAL CO., LTD., Tsukuba-shi, Ibaraki 3002611 (JP); KAWANISHI, Nobuhiko, BANYU PHARMACEUTICAL CO., LTD, Tsukuba-shi, Ibaraki 3002611 (JP); MITA, Takashi, BANYU PHARMACEUTICAL CO., LTD., Tsukuba-shi, Ibaraki 3002611 (JP); SHIMOMURA, Toshiyasu, BANYU PHARM. CO.,LTD., Tsukuba-shi, Ibaraki 3002611 (JP)
(74) Representative: Hussain, Deeba
(86) International application number: PCT/JP2005/019958
(87) International publication number: WO 2006/046735

(56) References cited:
- WO-A1-2006/008874
- WO-A2-02/057259
- WO-A2-02/059112
- JP-A- 2003 509 342

## Description

### BACKGROUND OF THE INVENTION

The present invention relates to novel aminopyridine derivatives which are useful in the pharmaceutical field, and more particularly, to those which inhibit the growth of tumor cells based on an Aurora A selective inhibitory action and exhibit an antitumor effect, and also to an Aurora A selective inhibitor and an antitumor agent containing them.

Aurora kinase is a serine/threonine kinase involved in cell division. With regard to the Aurora kinase, three subtypes of A, B and C are known at present, and they have very high homology to each other. Aurora A participates in the maturation and distribution of centrosome or in the formation of spindle body. On the other hand, it is believed that Aurora B participates in the aggregation and pairing of chromosome, a spindle checkpoint and cytoplasm division *[*Nat. Rev. Mol. Cell Biol., No. 4, pp. 842-854]. Also, it is believed that Aurora C acts similarly as a result of interaction with Aurora B *[*J. Biol. Chem., Epub ahead (2004)]. From the fact that high expression of Aurora A has been hitherto confirmed in many cancer cells; that high expression of Aurora A in normal cells leads to transformation of normal cell strains of rodent; and the like, Aurora A, being one of oncogenes, is recognized to be an adequate target for an antitumor agent *[*EMBO J., No. 17, pp. 3052-3065 (1998)].

There is another report that cancer cells in which Aurora A is highly expressed have a resistance to paclitaxel *[*Cancer Cell, Vol. 3, pp. 51-62 (2003)]. Meanwhile, with regard to the Aurora kinase inhibitor, development of subtype-selective drugs has been thought to be difficult in view of high homology among subtypes, protein structure analysis and the like; and although there have been known reports on drugs such as ZM447439 which inhibit both Aurora A and Aurora B at the same time *[*J. Cell Biol., No. 161, pp. 267-280 (2003); J. Cell Biol., No. 161, pp. 281-294, (2003); Nat. Med., No. 10, pp. 262-267, (2004)], no report concerning Aurora A selective drugs have been known. Thus, in those reports, disclosed is the antitumor effect only for the case where a drug which inhibits both Aurora A and Aurora B at the same time is solely administered. In addition, there has been also reported a result that in a drug which inhibits both Aurora A and Aurora B at the same time, the Aurora kinase inhibiting action attenuates the action of paclitaxel *[*J. Cell Biol., No. 161, pp. 281-294, (2003)].

Now, patent applications concerning compounds having an Aurora kinase inhibiting action have been previously filed (WO 02/057259, U.S. Patent No. 6,664,247, etc.), and patent applications concerning aminopyridine derivatives has been filed as well (U.S. Patent No. 6,586,424, etc.). However, there has been no report on an aminopyridine derivative having an excellent Aurora A selective inhibitory action thus far.
WO-A-02059112 (Vertex Pharmaceuticals Incorporated) discloses N-1H-pyrazolylpyridazinylamines and N-1H-pyrazolyltriazinylamines as inhibitors of Aurora 2 kinase.

### DETAILED DESCRIPTION OF THE INVENTION

The problems that the present invention should solve are to create novel aminopyridine derivatives which show an excellent Aurora A selective inhibitory action and cell-growth inhibitory action based on the foregoing, as well as achieve a synergistic action by a combined use with other antitumor agent(s).

In order to solve the above problems, the present inventors have synthesized a variety of novel aminopyridine derivatives and found that the compound represented by the following Formula (I) shows an excellent Aurora A selective inhibitory action and cell-growth inhibitory action based on the foregoing, and also achieves a synergistic action by a combined use with other antitumor agents, thus completing the invention. With regard to those cancers which have been unable to be completely treated with known antitumor agents such as paclitaxel because it has been impossible to use a sufficient amount of the agents owing to side-effects or drug resistance thereof, the administration of the compound according to the invention or the combined administration of the compound according to the invention with other antitumor agent is expected to exhibit an excellent antitumor effect (including potentiation of action due to the other antitumor agent) and an effect of attenuating side-effects.

Thus, the invention relates to a compound of general formula I: 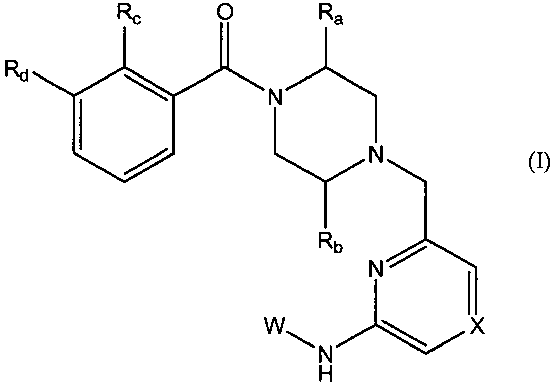 wherein:
Rₐ and R_{b} are each independently hydrogen atom or a linear or branched alkyl group having 1 to 6 carbon atoms, or alternatively Rₐ and R_{b} are combined together to form -CH₂-;
R_{c} is halogen atom;
R_{d} is halogen atom or methyl substituted with one to three halogen atoms;
X is CH, CX₁, or N wherein:
X₁ is CONR₁R₂ wherein R₁ and R₂ are each independently hydrogen atom, or lower alkyl which may be substituted;
W is selected from: 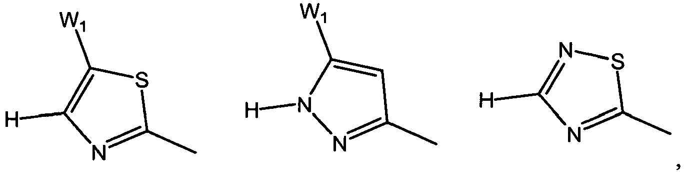 wherein W₁ is hydrogen atom, halogen atom, or methyl which may be substituted with one to three fluorine atoms;
   or a pharmaceutically acceptable salt thereof.

In another embodiment, the invention relates to a compound of general formula I: 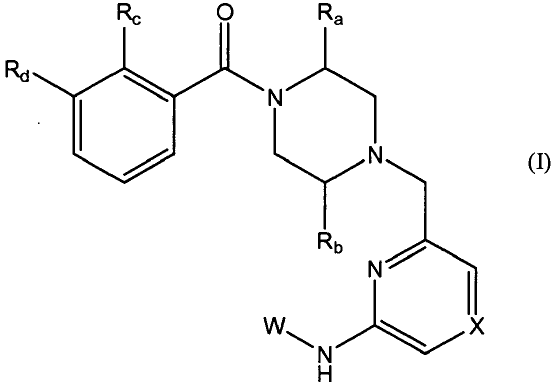 wherein:
Rₐ and R_{b} are each independently hydrogen atom or a linear or branched alkyl group having 1 to 6 carbon atoms, or alternatively Rₐ and R_{b} are combined together to form -CH₂-;
R_{c} is halogen atom;
R_{d} is halogen atom or methyl substituted with one to three halogen atoms;
X is CH or N; and
W is selected from: 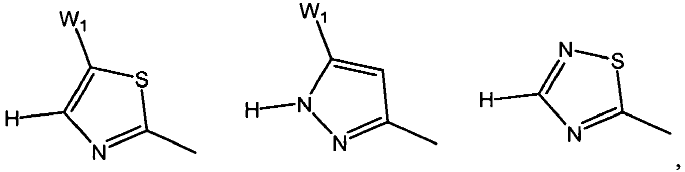 wherein W₁ is hydrogen atom, halogen atom, or methyl which may be substituted with one to three fluorine atoms; or agent in the field of pharmaceuticals. with the proviso that when W is 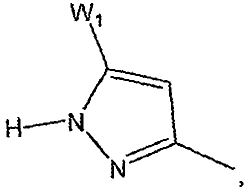 then X is N; or a pharmaceutically acceptable salt thereof.

The invention also relates to a combined preparation for simultaneous, separate or sequential administration in the treatment of cancer, comprising two separate preparations which are:
* a preparation comprising, together with a pharmaceutically acceptable carrier or diluent, a compound represented by the above-described Formula [I] or a pharmaceutically acceptable salt thereof; and
* a preparation comprising, together with a pharmaceutically acceptable carrier or diluent, one antitumor agent selected from the group consisting of antitumor alkylating agents, antitumor antimetabolites, antitumor antibiotics, plant-derived antitumor agents, antitumor platinum coordination compounds, antitumor camptothecin derivatives, antitumor tyrosine kinase inhibitors, monoclonal antibodies, interferons, biological response modifiers and other antitumor agents as well as pharmaceutically acceptable salt(s) or ester(s) thereof, wherein
the antitumor alkylating agent is nitrogen mustard N-oxide, cyclophosphamide, ifosfamide, melphalan, busulfan, mitobronitol; carboquone, thiotepa, ranimustine, nimustine, temozolomide or carmustin;
the antitumor antimetabolite is methotrexate, 6-mercaptopurine riboside, mercaptopurine, 5-fluorouracil, tegafur, doxyfluridine, carmofur, cytarabine, cytambine ocfosfate, enocitabine, S-1, gemcitabine, fludarabine or pemetrexed disodium;
the antitumor antibiotic is actinomycin D, doxorubicin, daunorubicin, neocarzinostatin, bleomycin, peplomycine, mitomycin C, aclarubicin, pirarubicin, epirubicin, zinostatin stimalamer, idarubicin, sirolimus or vairubicin;
the plant-derived antitumor agent is vincristine, vinblastine, vindesine, etoposide, sobuzoxane, docetaxel, paclitaxel or vinorelbine;
the antitumor platinum coordination compound is cisplatin, carboplatin, nedaplatin or oxaliplatin;
the antitumor camptothecin derivative is irinotecan, iopotecan or camptothecin;
the antitumor tyrosine kinase inhibitor is gefitinib, imatinib or erlotinib;
the monoclonal antibody is cetuximab, bevacizumab, rituximab, bevacizumab, alemtuzumab or trastuzumab;
the interferon is interferon α interferon α-2a, interferon α-2b, interferon β, interferon γ-1a or interferon γ-n1;
the biological response modifier is krestin, lentinan, sizofiran, picibanil or ubenimex; and
the other antitumor agent is mitoxantrone, L-asparaginase, procarbazine, dacarbazine, hydroxycarbamide, pentostatin, tretinoin, alefacept, darbepoetin alfa, anastrozole, exemestane, bicalutamide, leuprolelin, flutamide, fulvestrant, pegaptanib octasodium, denileukin diflitox, aldesleukin, thyrotropin alfa, arsenic trioxide, bortezomib, capecitabine or goserelin.

The invention further relates to a pharmaceutical composition comprising, together with a pharmaceutically acceptable carrier or diluent, a compound represented by the above-described Formula (I) or a pharmaceutically acceptable salt thereof, and an antitumor agent selected from the group consisting of antitumor allylating agents, antitumor antimetabolites, antitumor antibiotics, plant-derived antitumor agents, antitumor platinum coordination compounds, antitumor camptothecin derivatives, antitumor tyrosine kinase inhibitors, monoclonal antibodies, biological response modifiers and other antitumor agents (here, the definition of each antitumor agent is the same as that defined hereinabove) or a pharmaceutically acceptable salt or ester thereof.

Next, symbols and terms used in the present specification will be explained.

Examples of a linear or branched alkyl group having 1 to 6 carbon atoms include, for example, methyl, ethyl, propyl, isopropyl, butyl, isobutyl, sec-butyl, tert-butyl, pentyl and hexyl, among these methyl being preferred.

The term "halogen atom" in the above Formula (I) is, for example, fluorine atom, chlorine atom, bromine atom or iodine atom. Among them, for example, fluorine atom, chlorine atom or bromine atom is preferred; fluorine atom or chlorine atom is more preferred.

The term "selective inhibitor of Aurora A" used in the present specification is a compound or a drug which selectively inhibits Aurora A as compared with Aurora B.

Explanation for the term "pharmaceutically acceptable salt thereof" or the term "pharmaceutically acceptable carrier or diluent" used in the specification still will be given later.

The term "treatment of cancer" as used in the specification means inhibition of cancer cell growth by administering an antitumor agent to a cancer patient. Preferably, this treatment enables retrogression of cancer growth, that is, reduction in the measurable cancer size. More preferably, such treatment completely eliminates cancer.

The term "cancer" as used in the specification refers to solid cancer and hematopoietic cancer. Here, examples of solid cancer include cerebral tumor, head and neck cancer, esophageal cancer, thyroid cancer, small cell lung cancer, non-small cell lung cancer, breast cancer, stomach cancer, gallbladder and bile duct cancer, liver cancer, pancreas cancer, colon cancer, rectal cancer, ovarian cancer, chorioepithelioma, uterine cancer, cervical cancer, renal pelvic and ureteral cancer, bladder cancer, prostate cancer, penile cancer, testicular cancer, embryonal cancer, wilms tumor, skin cancer, malignant melanoma, neuroblastoma, osteosarcoma, Ewing's tumor and soft tissue sarcoma. On the other hand, examples of hematopoietic cancer include acute leukemia, chronic lymphatic leukemia, chronic myelocytic leukemia, polycythemia vera, malignant lymphoma, multiple myeloma and non-Hodgkins' lymphoma.

The term "preparation" as used in the specification includes oral preparations and parenteral preparations. Examples of oral preparations include tablets, capsules, powders and granules, while examples of parenteral preparations include sterilized liquid preparations such as solutions or suspensions, specifically injections or drip infusions. Preferably, they are intravenous injections or intravenous drip infusions, and more preferably intravenous drip infusions.

The term "combined preparation" as used in the specification refers to those comprising two or more preparations for simultaneous, separate or sequential administration in the treatment, and such preparation may be a so-called kit type preparation or pharmaceutical composition. The term "combined preparation" also includes those having one or more preparations further combined with the combined preparation comprising two separate preparations used in the treatment of cancer.

The two separate preparations described above can be further combined with, in combination with a pharmaceutically acceptable carrier or diluent, at least one preparation comprising at least one antitumor agent selected from the group consisting of antitumor alkylating agents, antitumor antimetabolites, antitumor antibiotics, plant-derived antitumor agents, antitumor platinum coordination compounds, antitumor camptothecin derivatives, antitumor tyrosine kinase inhibitors, monoclonal antibodies, interferons, biological response modifiers and other antitumor agents (here, definition of each antitumor agent is the same as that defined above), or a pharmaceutically acceptable salt or ester thereof. In this case, the above-mentioned at least one preparation that has been further combined can be administered simultaneously, separately or sequentially with respect to the two separate preparations. For example, a combined preparation comprising three preparations may include that is comprised of a preparation including a preparation containing the compound represented by the above Formula (I), a preparation containing 5-fluorouracil and a preparation containing leucovorin.

Here, in the above-mentioned combined preparation, either or both of the two separate preparations may be parenteral preparations, preferably injections or drip infusions, and more preferably intravenous drip infusions.

The term "preparation" according to the invention may usually comprise a therapeutically effective amount of a compound according to the invention, together with a pharmaceutically acceptable carrier or diluent. This technique of formulation is considered to be a technical common knowledge to those having ordinary skill in the pertinent art and is well known. Preferably, intravenous drip infusions or injections can be prepared in combination with a pharmaceutically acceptable carrier or diluent, by various methods that are well known in the art.

In the case of using the combined preparation according to the invention, the term "administration" as used in the present specification refers to parenteral administration and/or oral administration, and preferably parenteral administration. Thus, when a combined preparation is administered, both administrations may be parenteral; one administration may be parenteral while the other may be oral; or both administrations may be oral. Preferably, both preparations in the combined preparation are administered parenterally. Here, the term "parenteral administration" is, for example, intravenous administration, subcutaneous administration or intramuscular administration, and preferably it is intravenous administration. Even when three or more preparations are combined and administered, at least one preparation may be parenterally administered, preferably intravenously administered, and more preferably intravenously infused or intravenously injected.

In the embodiment of the present invention, a compound represented by the above Formula (I) may be administered simultaneously with other antitumor agent(s). Further, it is possible to administer the compound represented by the above Formula (I) first and then another antitumor agent consecutively, or alternatively it is possible to administer another antitumor agent first and then the compound represented by the above Formula (I) consecutively. It is also possible to administer the compound represented by the above Formula (I) first and then separately administer another antitumor agent after a while, or alternatively it is possible to administer another antitumor agent first and then separately administer the compound represented by the above Formula (I) after a while. The order and the time interval for the administration may be appropriately selected by a person skilled in the art in accordance with, for example, a preparation containing the compound represented by the above Formula (I) used and a preparation containing an antitumor agent that is used in combination therewith, the type of the cancer cells to be treated and the condition of the patient. For example, in the case of administering the compound represented by the above Formula (I) and paclitaxel, preferably paclitaxel is administered first, and then the compound represented by the above Formula (I) is administered sequentially or separately after a while.

The term "simultaneously" as used in the specification refers to the use of preparations for the treatment substantially at the same time, whereas the term "separately" refers to the separate use of preparations for the treatment at different times such that, for example, one agent is used on the first day and another agent is used on the second day for the treatment. The term "sequentially" refers to the use of preparations in such an order that, for example, one agent is first used and another agent is used after a predetermined period of time for the treatment.

The term "antitumor alkylating agent" as used in the present specification refers to an alkylating agent having antitumor activity, and the term "alkylating agent" herein generally refers to an agent giving an alkyl group in the alkylation reaction in which a hydrogen atom of an organic compound is substituted with an alkyl group. The term "antitumor alkylating agent" may be exemplified by nitrogen mustard N-oxide, cyclophosphamide, ifosfamide, melphalan, busulfan, mitobronitol, carboquone, thiotepa, ranimustine, nimustine, temozolomide or carmustine.

The term "antitumor antimetabolite" as used in the specification refers to an antimetabolite having antitumor activity, and the term "antimetabolite" herein includes, in a broad sense, substances which disturb normal metabolism and substances which inhibit the electron transfer system to prevent the production of energy-rich intermediates, due to their structural or functional similarities to metabolites that are important for living organisms (such as vitamins, coenzymes, amino acids and saccharides). The term "antitumor antimetabolites" may be exemplified methotrexate, 6-mercaptopurine riboside, mercaptopurine, 5-fluorouracil, tegafur, doxifluridine, carmofur, cytarabine, cytarabine ocfosfate, enocitabine, S-1, gemcitabine, fludarabine or pemetrexed disodium, and preferred are 5-fluorouracil, S-1, gemcitabine and the like.

The term "antitumor antibiotic" as used in the specification refers to an antibiotic having antitumor activity, and the "antibiotic" herein includes substances that are produced by microorganisms and inhibit cell growth and other functions of microorganisms and of other living organisms. The term "antitumor antibiotic" may be exemplified by actinomycin D, doxorubicin, daunorubicin, neocarzinostatin, bleomycin, peplomycin, mitomycin C, aclarubicin, pirarubicin, epirubicin, zinostatin stimalamer, idarubicin, sirolimus or valrubicin.

The term "plant-derived antitumor agent" as used in the specification includes compounds having antitumor activities which originate from plants, or compounds prepared by applying chemical modification to the foregoing compounds. The term "plant-derived antitumor agent" may be exemplified by vincristine, vinblastine, vindesine, etoposide, sobuzoxane, docetaxel, paclitaxel and vinorelbine, and preferred and docetaxel and paclitaxel.

The term "antitumor camptothecin derivative" as used in the specification refers to compounds that are structurally related to camptothecin and inhibit cancer cell growth, including camptothecin *per se.* The term "antitumor camptothecin derivative" is not particularly limited to, but may be exemplified by, camptothecin, 10-hydroxycamptothecin, topotecan, irinotecan or 9-aminocamptothecin, with camptothecin, topotecan and irinotecan being preferred. Further, irinotecan is metabolized in vivo and exhibits antitumor effect as SN-38. The action mechanism and the activity of the camptothecin derivatives are believed to be virtually the same as those of camptothecin (e.g., Nitta, et al., Gan to Kagaku Ryoho, 14, 850-857 (1987)).

The term "antitumor platinum coordination compound" as used in the specification refers to a platinum coordination compound having antitumor activity, and the term "platinum coordination compound" herein refers to a platinum coordination compound which provides platinum in ion form. Preferred platinum compounds include cisplatin; cis-diamminediaquoplatinum (II)-ion; chloro(diethylenetriamine)-platinum (II) chloride; dichloro(ethylenediamine)-platinum (II); diammine(1,1-cyclobutanedicarboxylato) platinum (II) (carboplatin); spiroplatin; iproplatin; diammine(2-ethylmalonato)platinum (II); ethylenediaminemalonatoplatinum (II); aqua(1,2-diaminodicyclohexane)sulfatoplatinum (II); aqua(1,2-diaminodicyclohexane)malonatoplatinum (II); (1,2-diaminocyclohexane)malonatoplatinum (II); (4-carboxyphthalato)(1,2-diaminocyclohexane) platinum (II); (1,2-diaminocyclohexane)-(isocitrato)platinum (II); (1,2-diaminocyclohexane)oxalatoplatinum (II); ormaplatin; tetraplatin; carboplatin, nedaplatin and oxaliplatin, and preferred is carboplatin or oxaliplatin. Further, other antitumor platinum coordination compounds mentioned in the specification are known and are commercially available and/or producible by a person having ordinary skill in the art by conventional techniques.

The term "antitumor tyrosine kinase inhibitor" as used in the specification refers to a tyrosine kinase inhibitor having antitumor activity, and the term "tyrosine kinase inhibitor" herein refers to a chemical substance inhibiting "tyrosine kinase" which transfers a γ-phosphate group of ATP to a hydroxyl group of a specific tyrosine in protein. The term "antitumor tyrosine kinase inhibitor" may be exemplified by gefitinib, imatinib or erlotinib.

The term "monoclonal antibody" as used in the specification, which is also known as single clonal antibody, refers to an antibody produced by a monoclonal antibody-producing cell, and examples thereof include cetuximab, bevacizumab, rituximab, alemtuzumab and trastuzumab.

The term "interferon" as used in the specification refers to an interferon having antitumor activity, and it is a glycoprotein having a molecular weight of about 20,000 which is produced and secreted by most animal cells upon viral infection. It has not only the effect of inhibiting viral growth but also various immune effector mechanisms including inhibition of growth of cells (in particular, tumor cells) and enhancement of the natural killer cell activity, thus being designated as one type of cytokine. Examples of "interferon" include interferon α, interferon α-2a, interferon α-2b, interferon β, interferon γ-1a and interferon γ-n1.

The term "biological response modifier" as used in the specification is the so-called biological response modifier or BRM and is generally the generic term for substances or drugs for modifying the defense mechanisms of living organisms or biological responses such as survival, growth or differentiation of tissue cells in order to direct them to be useful for an individual against tumor, infection or other diseases. Examples of the "biological response modifier" include krestin, lentinan, sizofiran, picibanil and ubenimex.

The term "other antitumor agent" as used in the specification refers to an antitumor agent which does not belong to any of the above-described agents having antitumor activities. Examples of the "other antitumor agent" include mitoxantrone, L-asparaginase, procarbazine, dacarbazine, hydroxycarbamide, pentostatin, tretinoin, alefacept, darbepoetin alfa, anastrozole, exemestane, bicalutamide, leuprorelin, flutamide, fulvestrant, pegaptanib octasodium, denileukin diftitox, aldesleukin, thyrotropin alfa, arsenic trioxide, bortezomib, capecitabine, and goserelin.

The above-described terms "antitumor alkylating agent", "antitumor antimetabolite", "antitumor antibiotic", "plant-derived antitumor agent", "antitumor platinum coordination compound", "antitumor camptothecin derivative", "antitumor tyrosine kinase inhibitor", "monoclonal antibody", "interferon", "biological response modifier" and "other antitumor agent" are all known and are either commercially available or producible by a person skilled in the art by methods known *per se* or by well-known or conventional methods. The process for preparation of gefitinib is described, for example, in USP No. 5,770,599; the process for preparation of cetuximab is described, for example, in WO 96/40210; the process for preparation of bevacizumab is described, for example, in WO 94/10202; the process for preparation of oxaliplatin is described, for example, in USP Nos. 5,420,319 and 5,959,133; the process for preparation of gemcitabine is described, for example, in USP Nos. 5,434,254 and 5,223,608; and the process for preparation of camptothecin is described in USP Nos. 5,162,532, 5,247,089, 5,191,082, 5,200,524, 5,243,050 and 5,321,140; the process for preparation of irinotecan is described, for example, in USP No. 4,604,463; the process for preparation of topotecan is described, for example, in USP No. 5,734,056; the process for preparation of temozolomide is described, for example, in JP-B No. 4-5029; and the process for preparation of rituximab is described, for example, in JP-W No. 2-503143.

The above-mentioned antitumor alkylating agents are commercially available, as exemplified by the following: nitrogen mustard N-oxide from Mitsubishi Pharma Corp. as Nitromin (tradename); cyclophosphamide from Shionogi & Co., Ltd. as Endoxan (tradename); ifosfamide from Shionogi & Co., Ltd. as Ifomide (tradename); melphalan from GlaxoSmithKline Corp. as Alkeran (tradename); busulfan from Takeda Pharmaceutical Co., Ltd. as Mablin (tradename); mitobronitol from Kyorin Pharmaceutical Co., Ltd. as Myebrol (tradename); carboquone from Sankyo Co., Ltd. as Esquinon (tradename); thiotepa from Sumitomo Pharmaceutical Co., Ltd. as Tespamin (tradename); ranimustine from Mitsubishi Pharma Corp. as Cymerin (tradename); nimustine from Sankyo Co., Ltd. as Nidran (tradename); temozolomide from Schering Corp. as Temodar (tradename); and carmustine from Guilford Pharmaceuticals Inc. as Gliadel Wafer (tradename).

The above-mentioned antitumor antimetabolites are commercially available, as exemplified by the following: methotrexate from Takeda Pharmaceutical Co., Ltd. as Methotrexate (tradename); 6-mercaptopurine riboside from Aventis Corp. as Thioinosine (tradename); mercaptopurine from Takeda Pharmaceutical Co., Ltd. as Leukerin (tradename); 5-fluorouracil from Kyowa Hakko Kogyo Co., Ltd. as 5-FU (tradename); tegafur from Taiho Pharmaceutical Co., Ltd. as Futraful (tradename); doxyfluridine from Nippon Roche Co., Ltd. as Furutulon (tradename); carmofur from Yamanouchi Pharmaceutical Co., Ltd. as Yamafur (tradename); cytarabine from Nippon Shinyaku Co., Ltd. as Cylocide (tradename); cytarabine ocfosfate from Nippon Kayaku Co., Ltd. as Strasid(tradename); enocitabine from Asahi Kasei Corp. as Sanrabin (tradename); S-1 from Taiho Pharmaceutical Co., Ltd. as TS-1 (tradename); gemcitabine from Eli Lilly & Co. as Gemzar (tradename); fludarabine from Nippon Schering Co., Ltd. as Fludara (tradename); and pemetrexed disodium from Eli Lilly & Co. as Alimta (tradename).

The above-mentioned antitumor antibiotics are commercially available, as exemplified by the following: actinomycin D from Banyu Pharmaceutical Co., Ltd. as Cosmegen (tradename); doxorubicin from Kyowa Hakko Kogyo Co., Ltd. as adriacin (tradename); daunorubicin from Meiji Seika Kaisha Ltd. as Daunomycin; neocarzinostatin from Yamanouchi Pharmaceutical Co., Ltd. as Neocarzinostatin (tradename); bleomycin from Nippon Kayaku Co., Ltd. as Bleo (tradename); pepromycin from Nippon Kayaku Co, Ltd. as Pepro (tradename); mitomycin C from Kyowa Hakko Kogyo Co., Ltd. as Mitomycin (tradename); aclarubicin from Yamanouchi Pharmaceutical Co., Ltd. as Aclacinon (tradename); pirarubicin from Nippon Kayaku Co., Ltd. as Pinorubicin (tradename); epirubicin from Pharmacia Corp. as Pharmorubicin (tradename); zinostatin stimalamer from Yamanouchi Pharmaceutical Co., Ltd. as Smancs (tradename); idarubicin from Pharmacia Corp. as Idamycin (tradename); sirolimus from Wyeth Corp. as Rapamune (tradename); and valrubicin from Anthra Pharmaceuticals Inc. as Valstar (tradename).

The above-mentioned plant-derived antitumor agents are commercially available, as exemplified by the following: vincristine from Shionogi & Co., Ltd. as Oncovin (tradename); vinblastine from Kyorin Pharmaceutical Co., Ltd. as Vinblastine (tradename); vindesine from Shionogi & Co., Ltd. as Fildesin (tradename); etoposide from Nippon Kayaku Co., Ltd. as Lastet (tradename); sobuzoxane from Zenyaku Kogyo Co., Ltd. as Perazolin (tradename); docetaxel from Aventis Corp. as Taxsotere (tadename); paclitaxel from Bristol-Myers Squibb Co. as Taxol (tradename); and vinorelbine from Kyowa Hakko Kogyo Co., Ltd. as Navelbine (tradename).

The above-mentioned antitumor platinum coordination compounds are commercially available, as exemplified by the following: cisplatin from Nippon Kayaku Co., Ltd. as Randa (tradename); carboplatin from Bristol-Myers Squibb Co. as Paraplatin (tradename); nedaplatin from Shionogi & Co., Ltd. as Aqupla (tradename); and oxaliplatin from Sanofi-Synthelabo Co. as Eloxatin (tradename).

The above-mentioned antitumor camptothecin derivatives are commercially available, as exemplified by the following: irinotecan from Yakult Honsha Co., Ltd. as Campto (tradename); topotecan from GlaxoSmithKline Corp. as Hycamtin (tradename); and camptothecin from Aldrich Chemical Co., Inc., U.S.A.

The above-mentioned antitumor tyrosine kinase inhibitors are commercially available, as exemplified by the following: gefitinib from AstraZeneca Corp. as Iressa (tradename); imatinib from Novartis AG as Gleevec (tradename); and erlotinib from OSI Pharmaceuticals Inc. as Tarceva (tradename).

The above-mentioned monoclonal antibodies are commercially available, as exemplified by the following: cetuximab from Bristol-Myers Squibb Co. as Erbitux (tradename); bevacizumab from Genentech, Inc. as Avastin (tradename); rituximab from Biogen Idec Inc. as Rituxan (tradename); alemtuzumab from Berlex Inc. as Campath (tradename); and trastuzumab from Chugai Pharmaceutical Co., Ltd. as Herceptin (tradename).

The above-mentioned interferons are commercially available, as exemplified by the following: interferon α from Sumitomo Pharmaceutical Co., Ltd. as Sumiferon (tradename); interferon α-2a from Takeda Pharmaceutical Co., Ltd. as Canferon-A (tradename); interferon α-2b from Schering-Plough Corp. as Intron A (tradename); interferon β from Mochida Pharmaceutical Co., Ltd. as IFNβ (tradename); interferon γ-1a from Shionogi & Co., Ltd. as Imunomax-γ (tradename); and interferon γ-n1 from Otsuka Pharmaceutical Co., Ltd. as Ogamma (tradename).

The above-mentioned biological response modifiers are commercially available, as exemplified by the following: krestin from Sankyo Co., Ltd. as krestin (tradename); lentinan from Aventis Corp. as Lentinan (tradename); sizofiran front Kaken Seiyaku Co., Ltd. as Sonifiran (tradename); picibanil from Chugai Pharmaceutical Co., Ltd. as Picibanil (tradename); and ubenimex from Nippon Kayaku Co., Ltd. as Bestatin (tradename).

The above-mentioned other antitumor agents are commercially available, as exemplified by the following: mitoxantrone from Wyeth Lederle Japan, Ltd. as Novantrone (tradename); L-asparaginase from Kyowa Hakko Kogyo Co., Ltd. as Leunase (tradename); procarbazine from Nippon Roche Co., Ltd. as Natulan (tradename); dacarbazine from Kyowa Hakko Kogyo Co., Ltd. as Dacarbazine (tradename); hydroxycarbamide from Bristol-Myers Squibb Co. as Hydrea (tradename); pentostatin from Kagaku Oyabi Kessei Ryoho Kenkyusho as Coforin (tradename); tretinoin from Nippon Roche Co., Ltd. As Vesanoid (tradename); alefacept from Biogen Idec Inc. as Amevive (tradename); darbepoetin alfa from Amgen Inc. as Aranesp (tradename); anastrozole from AstraZeneca Corp. as Arimidex (tradename); exemestane from Pfizer Inc. as Aromasin (tradename); bicalutamide from AstraZeneca Corp. as Casodex (tradename); leuprorelin from Takeda Pharmaceutical Co., Ltd. as Leuplin (tradename); flutamide from Schering-Plough Corp. as Eulexin (tradename); fulvestrant from AstraZeneca Corp. as Faslodex (tradename); pegaptanib octasodium from Gilead Sciences, Inc. as Macugen (tradename); denileukin diftitox from Ligand Pharmaceuticals Inc. as Ontak (tradename); aldesleukin from Chiron Corp. as Proleukin (tradename); thyrotropin alfa from Genzyme Corp. as Thyrogen (tradename); arsenic trioxide from Cell Therapeutics, Inc. as Trisenox (tradename); bortezomib from Millennium Pharmaceuticals, Inc. as Velcade (tradename); capecitabine from Hoffmann-La Roche, Ltd. as Xeloda (tradename); and goserclin from AstraZeneca Corp. as Zoladex (tradename).

Embodiments of the compound represented by the above General Formula (I) will be illustrated in more detail.

Rₐ and R_{b} are each independently hydrogen atom or a linear or branched alkyl group having 1 to 6 carbon atoms or alternatively Rₐ and R_{b} are combined to form -CH₂-. Preferably Rₐ and R_{b} are hydrogen atom.

R_{c} is halogen atom, preferably fluorine atom or chlorine atom, more preferably fluorine atom.

R_{d} is halogen atom or methyl substituted with one to three halogen atoms; preferably chlorine atom or trifluoromethyl.

X is CH, CX₁, or N, wherein:

X₁ is CONR₁R₂ wherein R₁ and R₂ are each independently hydrogen atom, or lower alkyl which may be substituted.

X is preferably CH or N.

W is selected from: 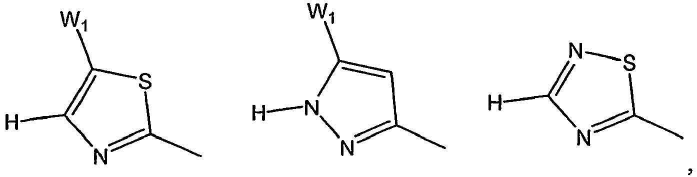 wherein W₁ is hydrogen atom, halogen atom, or methyl which may be substituted with one to three fluorine atoms.

W is preferably any of the following:

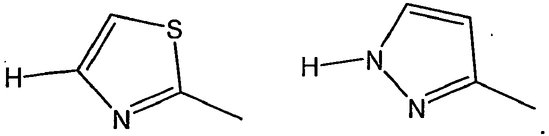

A preferred embodiment of the compound represented by the above General Formula (I) can be also expressed as follows:
(a) 6-((4-(3-chloro-2-fluorobenzoyl)piperazin-1-yl)methyl)-N-thiazol-2-ylpyridin-2-amine;
(b) 6-((4-(2,3-dichlorobenzoyl)piperazin-1-yl)methyl)-N-thiazol-2-ylpyridin-2-amine;
(c) 6-(((1S,4S)-5-(3-chloro-2-fluorobenzoyl)-2,5-diazabicyclo(2.2.1)hept-2-yl)methyl)-N-thiazol-2 -ylpyridin-2-amine;
(d) 6-((4-(3-chloro-2-fluorobenzoyl)piperazin-1-yl)methyl)-N-1H-pyrazol-3-ylpyridin-2-amine;
(e) 6-((4-(2-fluoro-3-(trifluoromethyl)benzoyl)piperazin-1-yl)methyl)-N-1H-pyrazol-3-ylpyridin-2-amine;
(f) 6-((4-(3-(difluoromethyl)-2-fluorobenzoyl)piperazin-1-yl)methyl)-N-1H-pyrazol-3-ylpyridin-2-amine;
(g) 6-((4-(3-chloro-2-fluorobenzoyl)piperazin-1-yl)methyl)-N-(5-methyl-1H-pyrazol-3-yl)pyridin-2-amine;
(h) 6-((4-(2-fluoro-3-(trifluoromethyl)benzoyl)piperazin-1-yl)methyl)-N-(5-methyl-1H-pyrazol-3-yl)pyridin-2-amine;
(i) 6-((4-(2-fluoro-3-(trifluoromethyl)benzoyl)piperazin-1-yl)methyl)-N-1,2,4-thiadiazol-5-yl pyridin-2-amine;
(j) 6-((4-(2-fluoro-3-(trifluoromethyl)benzoyl)piperazin-1-yl)methyl)-N-1H-pyrazol-3-ylpyrazin-2-amine;
(k) 6-(((1S,4S)-5-(3-chloro-2-fluorobenzoyl)-2,5-diazabicyclo(2.2.1)hept-2-yl)methyl)-N-1H-pyrazol-3-ylpyrazin-2-amine;
(l) 6-((4-(2-fluoro-3-(trifluoromethyl)benzoyl)piperazin-1-yl)methyl)-N-1H-pyrazol-3-ylpyrazin-2-amine;
(m) 6-((4-(3-chloro-2-fluorobenzoyl)piperazin-1-yl)methyl)-N-(5-methyl-1H-pyrazol-3-yl)pyrazin-2-amine;
(n) 6-((4-(3-chloro-2-fluorobenzoyl)piperazin-1-yl)methyl)-N-thiazol-2-ylpyrazin-2-amine;
(o) 6-((4-(3-chloroo-2-fluorobenzoyl)piperazin-1-yl)methyl)-N-(5-chlorothiazol-2-yl)pyridin-2-amine; or
(p) 6-((4-(2-fluorobenzoyl-3-triflluoromethyl)piperazin-1-yl)methyl)-N-(5-methyl-1H-pyrazol-3-yl) pyrazin-2-amine;
or a pharmaceutically acceptable salt thereof.

Further, in the combined preparation comprising two separate preparations according to the invention, preferably either or both of the two separate preparations are parenteral preparations, and more preferably either or both of the two separate preparations are injections or drip infusions.

The combined preparation comprising two separate preparations according to the invention is preferably such that one of the preparations is a preparation containing anyone of the above (a) to (p) or a pharmaceutically acceptable salt thereof, together with a pharmaceutically acceptable carrier or diluent; and
the other preparation is a preparation containing paclitaxel or docetaxel or a pharmaceutically acceptable salt or ester thereof, together with a pharmaceutically acceptable carrier or diluent.

Also, the pharmaceutical composition according to the invention preferably contains any one of the above (a) to (p) or a pharmaceutically acceptable salt thereof; and paclitaxel or docetaxel, or a pharmaceutically acceptable salt or ester thereof, together with a pharmaceutically acceptable carrier or diluent.

Next, the Aurora A and Aurora B inhibitory actions of the compound of General Formula (I) according to the invention will be explained below.

### Aurora A Inhibitory Action

### (1) Purification of Aurora A

cDNA of Aurora A having histidine tag fused at the amino terminal was integrated into an expression vector, which was then highly expressed in Escherichia coli BL21-CodonPlus(DE3)-RIL cells. The Escherichia coli cells were recovered and solubilized, and then the histidine-tagged Aurora A protein was adsorbed onto a nickel chelate column and eluted from the column with imidazole. The active fraction was desalted with a desalting column to give a pure enzyme.

### (2) Measurement of activity of Aurora A

For measurement of the activity of Aurora A, the substrate used was Kemptide Leu-Arg-Arg-Ala-Ser-Lys-Gly)(SEQ.ID.NO.: 1), a synthetic peptide purchased from Sigma-Aldrich, Inc. [Certificate of analysis (Upstate, Inc.)].

Reaction was conducted by a partial modification of a method by Upstate, Inc. [Kinase Profiler™ Assay Protocols]. The amount of the reaction liquid was 21.1 µL, and the composition of the reaction buffer (R2 buffer) was 50 mM Tris-hydrochloride buffer (pH 7.4)/15 mM magnesium acetate/0.2 mM ethylenediamine-N,N,N',N'-tetraacetate (EDTA). To this, purified Aurora A, 100 µM of a substrate peptide, 20 µM of unlabeled adenosine triphosphate (ATP) and 0.5 µCi of [γ-³³P] labeled ATP (2,500 Ci/mmole or more) were added, and the mixture was reacted at 30°C for 20 minutes. Then, 10 µL of 350 mM phosphate buffer was added to the reaction system to stop the reaction. The substrate peptide was adsorbed on a P81 paper filter 96-well plate and then washed with 130 mM phosphate buffer for several times. The radiation activity of the peptide was measured with a liquid scintillation counter. The [γ-³³P] labeled ATP was purchased from Amersham Biosciences Co., Ltd.

The compound to be tested was added to the reaction system such that a dilution series of the compound in dimethylsulfoxide was first prepared, and 1.1 µL of this solution was added. A control was provided by adding 1.1 µL of DMSO to the reaction system.

### Aurora B Inhibitory Action

### (1) Purification of Aurora B

cDNA of Aurora B having histidine tag fused at the amino terminal was integrated into an expression vector, which was then highly expressed in Escherichia coli BL21-CodonPlus(DE3)-RIL cells. The Escherichia coli cells were recovered and solubilized, and then the histidine-tagged Aurora A protein was adsorbed onto a nickel chelate column and eluted from the column with imidazole. The active fraction was desalted with a desalting column to give a pure enzyme.

### (2) Measurement of activity of Aurora B

For measurement of the activity of Aurora B, the substrate used was Kemptide (Leu-Arg-Arg-Ala-Ser-Leu-Gly) (SEQ.ID.NO.: 1), a synthetic peptide purchased from Sigma-Aldrich, Inc. [Certificate of analysis (Upstate, Inc.)].

Reaction was conducted by a partial modification of the method of activity measurement for Aurora A. The amount of the reaction liquid was 21.1 µL, and the composition of the reaction buffer (R2 buffer) was 50 mM Tris-hydrochloride buffer (pH 7.4)/15 mM magnesium acetate/0.2 mM ethylenediamine-N,N,N',N'-tetraacetate (EDTA). To this, purified Aurora B, 100 µM of a substrate peptide, 100 µM of unlabeled adenosine triphosphate (ATP) and 1 µCi of [γ-³³P] labeled ATP (2,500 Ci/mmole or more) were added, and the mixture was reacted at 30°C for 20 minutes. Then, 10 µL of 350 mM phosphate buffer was added to the reaction system to stop the reaction. The substrate peptide was adsorbed on a P81 paper filter 96-well plate and then washed with 130 mM phosphate buffer for several times. The radiation activity of the peptide was measured with a liquid scintillation counter. The [γ-³³P] labeled ATP was purchased from Amersham Biosciences Co., Ltd.

The compound to be tested was added to the reaction system such that a dilution series of the compound in dimethylsulfoxide was first prepared, and 1.1 µL of this solution was added. A control was provided by adding 1.1 µL of DMSO to the reaction system.

The compound according to the invention exhibits excellent Aurora A selective inhibitory activity, as shown in Table 1.

**[Table 1]**

| Example | Aurora A inhibitory action (IC₅₀, nM) | Aurora B inhibitory action (IC₅₀, nM) |
|---|---|---|
| Example 1 | 0.67 | 440 |
| Example 2 | 0.5 | 200 |
| Example 3 | 0.49 | 92 |
| Example 4 | 0.99 | 1900 |
| Example 5 | 1.8 | 1300 |
| Example 6 | 0.9 | 530 |
| Example 7 | 2.9 | 1500 |
| Example 8 | 1.1 | 770 |
| Example 9 | 0.97 | 590 |
| Example 10 | 0.89 | 1100 |
| Example 11 | 6.1 | 1400 |
| Example 12 | 2.2 | 5400 |
| Example 13 | 2.1 | 2900 |
| Example 14 | 4.8 | 9900 |
| Example 16 | 0.44 | 89 |

Next, the cell growth suppressive action of the compound of the General Formula (I) according to the invention will be explained below.

### Method for judging the pharmaceutical effect using cells

### a) Reagent

Fetal calf serum (FCS) was purchased from Moregate Biotech, and DMEM medium was purchased from Invitrogen Corp. WST-8 was purchased from Kishida Chemical Co., Ltd.

### b) Cells

Human cervical cancer cells (HeLa S3) were obtained from the American Type Culture Collection (ATCC).

### c) Method of judging the effect

Cells were suspended in a DMEM medium containing 10% FCS, and the cell suspension was dispensed to a 96-well plastic plate at a rate of 750 cells/100 microliters per well. The plate was incubated overnight in 5% CO₂-95% air at 37°C. A drug was subjected to graded dilution in dimethylsulfoxide and further diluted with a DMEM medium containing 10% FCS. Then, the dilution was dispensed to the plate on which cells had been disseminated, at a rate of 100 microliters per well. The plate was incubated for further three days in 5% CO₂-95% air at 37°C. Cell growth after incubation was measured by the WST-8 method (H. Tominaga, et al., Anal. Commun., 36, 47-50 (1999)). Here, the WST-8 method refers to a method in which 20 microliters of a WST-8 reagent solution is added to each well, incubation is conducted at 37°C for 45 minutes, the plate is stirred, and the amount of formazan produced is measured by a colorimetric method to determine the inhibitory rate of the drug. The concentration for 50% growth inhibition (EC₅₀, µM) of the compound was determined.

The compound according to the invention exhibits excellent cell growth inhibitory effect against human-derived cancer cells (HeLa S3), as shown in Table 2.

**[Table 21**

| Example | Cell growth inhibitory effect (IC₅₀, µM) |
|---|---|
| Example 1 | 11.00 |
| Example 2 | 0.40 |
| Example 4 | 6.50 |
| Example 6 | 0.92 |
| Example 7 | 3.50 |
| Example 8 | 0.80 |
| Example 9 | 3.30 |
| Example 10 | 2.40 |
| Example 11 | 4.10 |
| Example 12 | 3.60 |
| Example 13 | 1.40 |
| Example 14 | 3.00 |
| Example 16 | 11.00 |

### Method for judging the effect by combined use of drugs in cells

### a) Reagent

Fetal calf serum (FCS) was purchased from Moregate Biotech, DMEM medium from Invitrogen Corp., paclitaxel (tradename: Taxol) from Sigma-Aldrich, Inc., and WST-8 from Kishida Chemical Co., Ltd.

### b) Cells

Human cervical cancer cells (HeLa S3) were obtained from the American Type Culture Collection (ATCC).

### c) Method of judging the effect

Cells were suspended in a DMEM medium containing 10% FCS, and the cell suspension was dispensed to two 96-well plastic plates at a rate of 750 cells/100 microliters per well. The plates were incubated overnight in 5% CO₂-95% air at 37°C. A drug was subjected to graded dilution in dimethylsulfoxide and further diluted with DMSO or with a DMEM medium containing 10% FCS and also containing 2 nM paclitaxel. Then, the dilutions were each dispensed to one of the plates on which cells had been disseminated, at a rate of 100 microliters per well. The final concentration of paclitaxel at this stage was 1 nM. Also, the concentrations in the case of sole administration of the compound according to the invention were 0.03, 0.1, 0.3, 1 and 3 µM. The plates were incubated for further three days in 5% CO₂-95% air at 37°C. Cell growth after incubation was measured by the WST-8 method (H. Tominaga, et al., Anal. Commun., 36, 47-50 (1999)). Here, the WST-8 method refers to a method in which 20 microliters of a WST-8 reagent solution is added to each well, incubation is conducted at 37°C for 45 minutes, the plate is stirred, and the amount of formazan produced is measured by a colorimetric method to determine the inhibitory rate of the drug. The growth inhibitory effects of paclitaxel and of the compound according to the invention were determined, with the value obtained in sole treatment of DMSO being defined as 0%.

The compound according to the invention exhibits excellent cell growth inhibitory effect as well as a synergistic action with paclitaxel against human-derived cancer cells (HeLa S3), as shown in Table 3.

**[Table 3]**

| Example | Cell growth inhibitory effect by sole administration of paclitaxel (1 nM) (%) | Conc. of the compound of Example (µM) | Cell growth inhibitory effect by sole administration of the compound of Example (%) | Cell growth inhibitory effect by combined administration of paclitaxel and the compound of Example (%) |
|---|---|---|---|---|
| Example 1 | 44.1 | 0.1 | 0.0 | 72.8 |
| Example 2 | 44.1 | 0.3 | 19.6 | 89.0 |
| Example 4 | 43.3 | 1.0 | 6.5 | 76.9 |
| Example 6 | 44.1 | 0.1 | 0.0 | 77.6 |
| Example 7 | 43.3 | 1.0 | 18.5 | 80.9 |
| Example 8 | 45.4 | 0.1 | 0.0 | 65.1 |
| Example 9 | 45.4 | 0.3 | 0.0 | 77.3 |
| Example 10 | 44.1 | 0.3 | 7.1 | 86.5 |
| Example 11 | 36.8 | 1.0 | 8.5 | 75.0 |
| Example 12 | 45.4 | 1.0 | 6.0 | 82.0 |
| Example 13 | 37.8 | 0.3 | 6.5 | 81.8 |
| Example 14 | 45.4 | 1.0 | 0.0 | 81.4 |
| Example 16 | 45.4 | 0.3 | 27.1 | 91.5 |

From the above, the compound according to the invention is believed to be useful as an antitumor agent since it exhibits not only excellent cell growth inhibitory action based on Aurora A selective inhibitory activity, but also a synergistic action in combined use with other antitumor agent. Thus, it is believed that a pharmaceutical composition or Aurora A selective inhibitor containing the novel aminopyridine derivative according to the invention or a pharmaceutically acceptable salt or ester thereof, or an antitumor agent containing the compound according to the invention or a pharmaceutically acceptable salt or ester thereof is effective in the treatment of cancer patients.

The above-mentioned pharmaceutical composition and inhibitor, and the above-mentioned antitumor agent may contain a pharmaceutically acceptable carrier or diluent. Here, the "pharmaceutically acceptable carrier or diluent" refers to excipients [e.g., fats, beeswax, semi-solid and liquid polyols, natural or hydrogenated oils, etc.]; water (e.g., distilled water, particularly distilled water for injection, etc.), physiological saline, alcohol (e.g., ethanol), glycerol, polyols, aqueous glucose solution, mannitol, plant oils, etc.); additives [e.g., extending agent, disintegrating agent, binder, lubricant, wetting agent, stabilizer, emulsifier, dispersant, preservative, sweetener, colorant, seasoning agent or aromatizer, concentrating agent, diluent, buffer substance, solvent or solubilizing agent, chemical for achieving storage effect, salt for modifying osmotic pressure, coating agent or antioxidant], and the like.

A suitable tumor for which the therapeutic effect of the compound according to the invention is expected may be exemplified by human solid cancer. Examples of human solid cancer include brain cancer, head and neck cancer, esophageal cancer, thyroid cancer, small cell carcinoma, non-small cell carcinoma, breast cancer, stomach cancer, gallbladder and bile duct cancer, liver cancer, pancreas cancer, colon cancer, rectal cancer, ovarian cancer, chorioepithelioma, uterine cancer, cervical cancer, renal pelvic and ureteral cancer, bladder cancer, prostate cancer, penile cancer, testicular cancer, embryonal cancer, Wilms' tumor, skin cancer, malignant melanoma, neuroblastoma, osteosarcoma, Ewing's tumor, soft tissue sarcoma, and the like.

Next, the above-described "pharmaceutically acceptable salt " will be explained below.

When the compound according to the invention is used as an antitumor agent or the like, it may be also used in a form of pharmaceutically acceptable salt. Typical examples of the pharmaceutically acceptable salt include a salt with an alkali metal such as sodium and potassium; a salt with an inorganic acid, such as hydrochloride, sulfate, nitrate, phosphate, carbonate, hydrogen carbonate, and perchlorate; a salt with an organic acid, such as acetate, propionate, lactate, maleate, fumarate, tartrate, malate, citrate, and ascorbate; a salt with sulfonic acid, such as methanesulfonate, isethionate, benzenesulfonate, and toluenesulfonate; a salt with acidic amino acid, such as aspartate and glutamate; and the like.

The process for preparation of a pharmaceutically acceptable salt of the compound according to the invention may be carried out by an appropriate combination of those methods that are conventionally used in the field of organic synthetic chemistry. A specific example thereof is a method in which a solution of the compound according to the invention in its free form is subjected to neutralization titration with an alkaline solution or an acidic solution.

With regard to each preparation of the combined preparation according to the invention, various preparation forms can be selected, and examples thereof include oral preparations such as tablets, capsules, powders, granules or liquids, or sterilized liquid parenteral preparations such as solutions or suspensions, suppositories, ointments and the like.

Solid preparations can be prepared in the forms of tablet, capsule, granule and powder without any additives, or prepared using appropriate carriers (additives). Examples of such carriers (additives) may include saccharides such as lactose or glucose; starch of corn, wheat or rice; fatty acids such as stearic acid; inorganic salts such as magnesium metasilicate aluminate or anhydrous calcium phosphate; synthetic polymers such as polyvinylpyrrolidone or polyalkylene glycol; alcohols such as stearyl alcohol or benzyl alcohol; synthetic cellulose derivatives such as methylcellulose, carboxymethylcellulose, ethylcellulose or hydroxypropylmethylcellulose; and other conventionally used additives such as gelatin, talc, plant oil and gum arabic.

These solid preparations such as tablets, capsules, granules and powders may generally contain, for example, 0.1 to 100% by weight, and preferably 5 to 98% by weight, of the compound of the above Formula (I) as an active ingredient, based on the total weight of the preparation.

Liquid preparations are produced in the forms of suspension, syrup, injection and drip infusion (intravenous fluid) using appropriate additives that are conventionally used in liquid preparations, such as water, alcohol or a plant-derived oil such as soybean oil, peanut oil and sesame oil.

In particular, when the preparation is administered parenterally in a form of intramuscular injection, intravenous injection or subcutaneous injection, appropriate solvent or diluent may be exemplified by distilled water for injection, an aqueous solution of lidocaine hydrochloride (for intramuscular injection), physiological saline, aqueous glucose solution, ethanol, polyethylene glycol, propylene glycol, liquid for intravenous injection (e.g., an aqueous solution of citric acid, sodium citrate and the like) or an electrolytic solution (for intravenous drip infusion and intravenous injection), or a mixed solution thereof.

Such injection may be in a form of a preliminarily dissolved solution, or in a form of powder per *se* or powder associated with a suitable carrier (additive) which is dissolved at the time of use. The injection liquid may contain, for example, 0.1 to 10% by weight of an active ingredient based on the total weight of the preparation.

Liquid preparations such as suspension or syrup for oral administration may contain, for example, 0.1 to 10% by weight of an active ingredient based on the total weight of the preparation.

Each preparation of the combined preparation according to the invention can be prepared by a person having ordinary skill in the art according to conventional methods or common techniques. For example, a preparation containing another antitumor agent that is used in combination with the compound represented by the above General Formula (I), can be prepared, if the preparation is an oral preparation, for example, by mixing an appropriate amount of the antitumor agent with an appropriate amount of lactose and filling this mixture into hard gelatin capsules which are suitable for oral administration. On the other hand, preparation can be carried out, if the preparation containing the antitumor agent is an injection, for example, by mixing an appropriate amount of the antitumor agent with an appropriate amount of 0.9% physiological saline and filling this mixture in vials for injection.

Also, in the case of a combination preparation containing the compound represented by the above General Formula (I) according to the invention and another antitumor agent, a person having ordinary skill in the art can easily prepare the preparation according to conventional methods or common techniques.

In the process according to the invention, preferred therapeutic unit may vary in accordance with, for example, the administration route of the compound represented by the General Formula (I), the type of the compound represented by the General Formula (I) used, and the dosage form of the compound represented by the General Formula (I) used; the type, administration route and dosage form of the other antitumor agent used in combination; and the type of cells to be treated, the condition of patient, and the like. The optimal treatment under the given conditions can be determined by a person skilled in the art, based on the set conventional therapeutic unit and/or based on the content of the present specification.

In the process according to the invention, the therapeutic unit for the compound represented by the above General Formula (I) may vary in accordance with, specifically, the type of compound used, the type of compounded composition, application frequency and the specific site to be treated, seriousness of the disease; age of the patient, doctor's diagnosis, the type of cancer, or the like. However, as an exemplary reference, the daily dose for an adult may be within a range of, for example, 1 to 1,000 mg in the case of oral administration. In the case of parenteral administration, preferably intravenous administration, and more preferably intravenous drip infusion, the daily dose may be within a range of, for example, 1 to 100 mg/m² (body surface area). Here, in the case of intravenous drip infusion, administration may be continuously carried out for, for example, 1 to 48 hours. Moreover, the administration frequency may vary depending on the administering method and symptoms, but it is, for example, once to five times a day. Alternatively, periodically intermittent administration such as administration every other day, administration every two days or the like may be employed as well in the administering method. The period of withdraw from medication in the case of parenteral administration is, for example, 1 to 6 weeks.

Although the therapeutic unit for the other antitumor agent used in combination with the compound represented by the General Formula (I) is not particularly limited, it can be determined, if needed, by those skilled in the art according to known literatures. Examples may be as follows.

The therapeutic unit of 5-fluorouracil (5-FU) is such that, in the case of oral administration, for example, 200 to 300 mg per day is administered in once to three times consecutively, and in the case of injection, for example, 5 to 15 mg/kg per day is administered once a day for the first 5 consecutive days by intravenous injection or intravenous drip infusion, and then 5 to 7.5 mg/kg is administered once a day every other day by intravenous injection or intravenous drip infusion (the dose may be appropriately increased or decreased).

The therapeutic unit of S-1 (Tegafur, Gimestat and Ostat potassium) is such that, for example, the initial dose (singe dose) is set to the following standard amount in accordance with the body surface area, and it is orally administered twice a day, after breakfast and after dinner, for 28 consecutive days, followed by withdrawal from medication for 14 days. This is set as one course of administration, which is repeated. The initial standard amount per unit body surface area (Tegafur equivalent) is 40 mg in one administration for an area less than 1.25 m²; 50 mg in one administration for an area of 1.25 m² to less than 1.5 m²; 60 mg in one administration for an area of 1.5 m² or more. This dose is appropriately increased or decreased depending on the condition of the patient.

The therapeutic unit for gemcitabine is, for example, 1 g as gemcitabine/m² in one administration, which is administered by intravenous drip infusion over a period of 30 minutes, and one administration per week is continued for 3 weeks, followed by withdrawal from medication on the fourth week. This is set as one course of administration, which is repeated. The dose is appropriately decreased in accordance with age, symptom or development of side-effects.

The therapeutic unit for doxorubicin (e.g., doxorubicin hydrochloride) is such that, for example, in the case of intravenous injection, 10 mg (0.2 mg/kg) (titer) is administered once a day by intravenous one-shot administration for 4 to 6 consecutive days, followed by withdrawal from medication for 7 to 10 days. This is set as one course of administration, which is repeated two or three times. Here, the total dose is preferably 500 mg (titer)/m² (body surface area) or less, and it may be appropriately increased or decreased within the range.

The therapeutic unit for etoposide is such that, for example, in the case of intravenous injection, 60 to 100 mg/m² (body surface area) per day is administered for 5 consecutive days, followed by withdrawal from medication for three weeks (the dose may be appropriately increased or decreased). This is set as one course of administration, which is repeated. Meanwhile, in the case of oral administration, for example, 175 to 200 mg per day is administered for 5 consecutive days, followed by withdrawal from medication for three weeks (the dose may be appropriately increased or decreased). This is set as one course of administration, which is repeated.

The therapeutic unit for docetaxel (docetaxel hydrate) is such that, for example, 60 mg as docetaxel/m² (body surface area) is administered once a day by intravenous drip infusion over a period of 1 hour or longer at an interval of 3 to 4 weeks (the dose may be appropriately increased or decreased).

The therapeutic unit of paclitaxel is such that, for example, 210 mg/m² (body surface area) is administered once a day by intravenous drip infusion over a period of 3 hours, followed by withdrawal from medication for at least 3 weeks. This is set as one course of administration, which is repeated. The dose may be appropriately increased or decreased.

The therapeutic unit for cisplatin is such that, for example, in the case of intravenous injection, 50 to 70 mg/m² (body surface area) is administered once a day, followed by withdrawal from medication for 3 weeks or longer (the dose may be appropriately increased or decreased). This is set as one course of administration, which is repeated.

The therapeutic unit for carboplatin is such that, for example, 300 to 400 mg/m² is administered once a day by intravenous drip infusion over a period of 30 minutes or longer, followed by withdrawal from medication for at least 4 weeks (the dose may be appropriately increased or decreased). This is set as one course of administration, which is repeated.

The therapeutic unit for oxaliplatin is such that 85 mg/m² is administered once a day by intravenous injection, followed by withdrawal from medication for two weeks. This is set as one course of administration, which is repeated.

The therapeutic unit for irinotecan (e.g., irinotecan hydrochloride) is such that, for example, 100 mg/m² is administered once a day by intravenous drip infusion for 3 or 4 times at an interval of one week, followed by withdrawal from medication for at least two weeks.

The therapeutic unit for topotecan is such that, for example, 1.5 mg/m² is administered once a day by intravenous drip infusion for 5 days, followed by withdrawal from medication for at least 3 weeks.

The therapeutic unit for cyclophosphamide is such that, for example, in the case of intravenous injection, 100 mg is administered once a day by intravenous injection for consecutive days. If the patient can tolerate, the daily dose may be increased to 200 mg. The total dose is 3,000 to 8,000 mg, which may be appropriately increased or decreased. If necessary, it may be injected or infused intramuscularly, intrathoracically or intratumorally. On the other hand, in the case of oral administration, for example, 100 to 200 mg is administered a day.

The therapeutic unit for gefitinib is such that 250 mg is orally administered once a day.

The therapeutic unit for cetuximab is such that, for example, 400 mg/m² is administered on the first day by intravenous drip infusion, and then 250 mg/m² is administered every week by intravenous drip infusion.

The therapeutic unit for bevacizumab is such that, for example, 3 mg/kg is administered every week by intravenous drip infusion.

The therapeutic unit for trastuzumab is such that, for example, typically for an adult, once a day, 4 mg as trastuzumab/kg (body weight) is administered initially, followed by intravenous drip infusion of 2 mg/kg over a period of 90 minutes or longer every week from the second administration.

The therapeutic unit for exemestane is such that, for example, typically for an adult, 25 mg is orally administered once a day after meal.

The therapeutic unit for leuprorelin (e.g., leuprorelin acetate) is such that, for example, typically for an adult, 11.25 mg is subcutaneously administered once in 12 weeks.

The therapeutic unit for imatinib is such that, for example, typically for an adult in the chronic phase of chronic myelogenous leukemia, 400 mg is orally administered once a day after meal.

The therapeutic unit for a combination of 5-FU and leucovorin is such that, for example, 425 mg/m² of 5-FU and 200 mg/m² of leucovorin are administered from the first day to the fifth day by intravenous drip infusion, and this course is repeated at an interval of 4 weeks.

### Examples

In a thin-layer chromatography of Examples and Referential Examples, Silica gel₆₀F₂₅₄ (Merck) was used as a plate and a UV detector was used in a detecting method. As silica gel for the column, Wakogel^{™} C-300 or C-200 (Wako Pure Chemical) or NH (FUJI SILYSIA CHEMICAL) was used. In a reversed phase preparative liquid chromatography, CombiPrep Pro C18 (YMC) was used as a column and a 0.1% aqueous trifluoroacetic acid solution and a 0.1% solution of trifluoroacetic acid in acetonitrile were used in a mobile phase. MS spectra were measured using JMS-SX102A (JEOL) or QUATTROII (Micro Mass). NMR spectra were measured using a spectrometer in a type of Gemini-200 (200 MHz; Varian), Gemini-300 (300 MHz; Varian), VXR-300 (300 MHz; Varian), Mercury 400 (400 MHz; Varian) or Inova 400 (400 MHz; Varian) and all δ values are represented in ppm.

Meanings of abbreviations used in the NMR measurement are as follows.
- s:: singlet
- d:: doublet
- dd:: double doublet
- t:: triplet
- dt:: double triplet
- q:: quartet
- qui:: quintet
- m:: multiplet
- br:: broad
- J:: coupling constant
- Hz:: Hertz
- DMSO-d₆:: dimethylsulfoxide-d₆
- TBS:: tert-butyldimethylsilyl group
- Ms:: methanesulfonyl group
- SEM:: 2-(trimethylsilyl)ethoxymethyl group
- MOM:: methoxymethyl group
- THP:: tetrahydropyran-2-yl group
- Boc:: tert-butoxycarbonyl group

### Example 1

### Synthesis of 6-((4-(3-chloro-2-fluorobenzoyl)piperazin-1-yl)methyl)-N-thiazol-2-ylpyridin-2-amine

### (1) Synthesis of 2-bromo-6-(((tert-butyl(dimethyl)silyl)oxy)methyl)pyridine

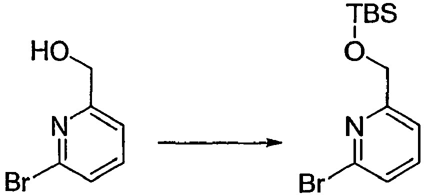

5.00 g of (6-bromo-pyridin-2-yl)-methanol was dissolved in 50 mL of dimethylformamide and 2.72 g of imidazole was added thereto. Under cooling with ice, 5.21 g of tert-butyldimethylsilyl chloride was added thereto followed by stirring at room temperature for 1 hour. The reaction solution was diluted with ethyl acetate and washed with water and brine. The resulting organic layer was dried over magnesium sulfate and filtered, and the filtrate was concentrated. The resulting residue was purified by a silica gel column chromatography (eluent: hexane/ethyl acetate = 10/1 to 1/1) to give the title compound.

### (2) Synthesis of 6-(((tert-butyl(dimethyl)silyl)oxy)methyl)-N-thiazol-2-ylpyridin-2-amine

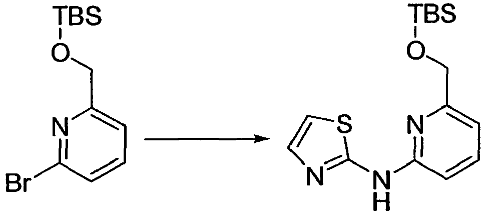

A mixture of 7.85 g of 2-bromo-6-(((tert-butyl(dimethyl)silyl)oxy)methyl)pyridine, 3.12 g of 2-aminothiazole, 1.50 g of 9,9-dimethyl-4,5-bis(diphenylphosphino)xanthene, 1.35 g of tris(dibenzylideneacetone)dipalladium(0)-chloroform complex, 13.8 g of potassium phosphate and 80 ml of 1,4-dioxane was stirred at 100°C for 4.5 hours, cooled to room temperature and diluted with ethyl acetate. An insoluble matter was filtered off using Celite and the resulting ethyl acetate solution was washed with water and brine. The organic layer was dried over anhydrous magnesium sulfate and filtered, and the filtrate was concentrated. The residue was purified by a silica gel column chromatography (eluent: hexane/ethyl acetate = 10/1 to 1/1) to give the title compound.

### (3) Synthesis of (6-(thiazol-2-ylamino)pyridin-2-yl)methanol

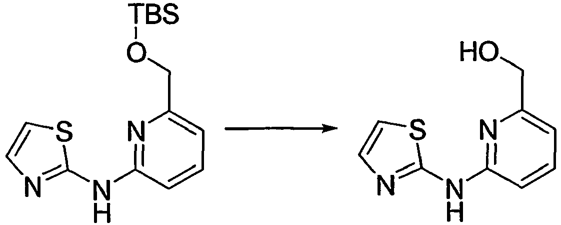

6.48g of 6-(((tert-butyl(dimethyl)silyl)oxy)methyl)-N-thiazol-2-ylpyridin-2-amine was dissolved in 100ml of tetrahydrofuran. Under cooling with ice, a tetrabutylammonium fluoride-tetrahydrofuran solution (1.0 M, 20.2 ml) was added followed by stirring at room temperature for 1 hour. The reaction solution was diluted with ethyl acetate and then washed with phosphate buffer (pH 6.8). The organic layer was dried over anhydrous magnesium sulfate and filtered, and the filtrate was concentrated to give the title compound.

### (4) Synthesis of 6-(chloromethyl)-N-thiazol-2-ylpyridin-2-amine

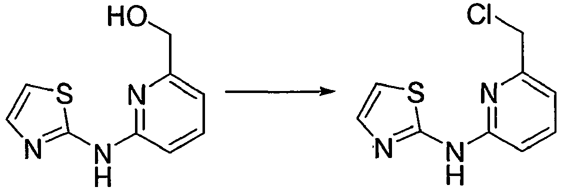

The entire amount of (6-(thiazol-2-ylamino)pyridin-2-yl)methanol obtained by the above operation was suspended in 150 ml of chloroform, and 7.37 ml of thionyl chloride was added thereto. After stirring at room temperature for 2 hours, the reaction solution was concentrated. The resulting residue was diluted with ethyl acetate, and then washed with a 2 M aqueous sodium hydroxide solution and brine. The organic layer was dried over anhydrous magnesium sulfate and filtered, and the filtrate was concentrated. The residue was purified by a silica gel column chromatography (eluent: chloroform to chloroform/methanol = 20/1) to give the title compound.

### (5) Synthesis of 6-((4-(3-chloro-2-fluorobenzoyl)piperazin-1-yl)methyl)-N-thiazol-2-ylpyridin-2-amine

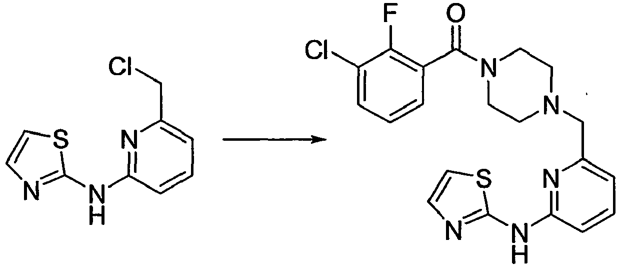

A mixture of 2.70 g of 6-(chloromethyl) N-thiazol-2-ylpyridin-2-amine, 4.00 g of 1-(3-chloro-2-fluorobenzoyl)piperazine obtained in Reference Example 1, 6.25 ml of N,N-diisopropylethylamine and 30 ml of dimethylformamide was stirred at 90°C for 2 hours. The reaction solution was diluted with ethyl acetate and then washed with water and brine. The resulting organic layer was dried over magnesium sulfate and filtered, and the filtrate was concentrated. The resulting residue was purified by a silica gel column chromatography (eluent: chloroform to chloroform/methanol = 10/1). Then, the obtained compound was suspended in ethyl acetate, and filtered and collected to give the title compound as a colorless amorphous substance.

Spectral data of the title compound are as follows.
¹H-NMR (DMSO-d₆) δ: 11.20 (s, 1H), 7.65 (t, J = 7.8 Hz, 2H), 7.41-7.33 (m, 2H), 7.29 (t, J = 7.8 Hz, 1H), 7.00-6.87 (m, 3H), 3.72-3.61 (m, 4H), 3.27-3.20 (m, 2H), 2.60-2.36 (m, 4H).
Mass: 432 (M+1)⁺

Examples 2 to 4 were synthesized in the same manner as in Example 1 as follows.

### Example 2

### Synthesis of 6-((4-(2,3-dichlorobenzoyl)piperazin-1-yl)methyl)-N-thiazol-2-ylpyridin-2-amine

¹H-NMR (DMSO-d₆) δ: 11.21 (s, 1H), 7.72-7.61 (m, 2H), 7.42 (t, J = 7.8 Hz, 1H), 7.39-7.29 (m, 2H), 7.02-6.90 (m, 3H), 3.73-3.61 (m, 4H), 3.19-3.12 (m, 2H), 2.60-2.38 (m, 4H)
Mass: 448 (M + 1)⁺

### Example 3

### Synthesis of 6-(((1S,4S)-5-(3-chloro-2-fluorobenzoyl)-2,5-diazabicyclo[2.2.1]hept-2-yl)methyl)-N-thiazol-2-ylpyridin-2-amine trifluoroacetate

¹H-NMR (DMSO-d₆) δ: 7.84-7.71 (m, 2H), 7.48-7.40 (m, 2H), 7.38-7.30 (m, 1H), 7.19-7.07 (m, 3H), 5.00-3.36 (m, 8H), 2.54-2.14 (m, 2H)
Mass: 444 (M + 1)⁺

### Example 4

### Synthesis of 6-((4-(2-fluoro-3-(trifluoromethyl)benzoyl)piperazin-1-yl)methyl)-N-1,2,4-thiadiazol-5-ylpyridin-2-amine

¹H-NMR (DMSO-d₆) δ: 12.18 (brs, 1H), 8.30 (s, 1H), 7.83-7.75 (m, 3H), 7.55-7.45 (m, 1H), 7.13 (d, J = 7.3 Hz, 1H), 7.05 (d, J = 8.3 Hz, 1H), 3.78-3.65 (m, 4H), 3.32-3.22 (m, 2H), 2.62-2.52 (m, 2H), 2.51-2.43 (m, 2H)
Mass: 467 (M + 1)⁺

### Example 5

### Synthesis of 6-((4-(3-chloro-2-fluorobenzoyl)piperazin-1-yl)methyl)-N-1H-pyrazol-3-ylpyridin-2-amine

### (1) Synthesis of (6-((1-((2-(trimethylsilyl)ethoxy)methyl)-1H-pyrazol-3-yl)amino)pyridin-2-yl)methanol

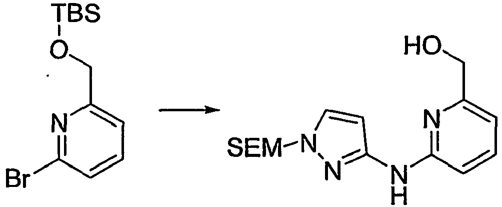

In the same manner as in Example 1-(2) to (3), the title compound was obtained using 2-bromo-6-(((tert-butyl(dimethyl)silyl)oxy)methyl)pyridine obtained in Example 1-(1) and 1-((2-(trimethylsilyl)ethoxy)methyl)-1-H-pyrazol-3-amine obtained in Reference Example 2.

### (2) Synthesis of 6-(methanesulfonyloxymethyl)-N-(1-((2-(trimethylsilyl)ethoxy)methyl)-1H-pyrazol-3 - yl)pyridin-2-amine

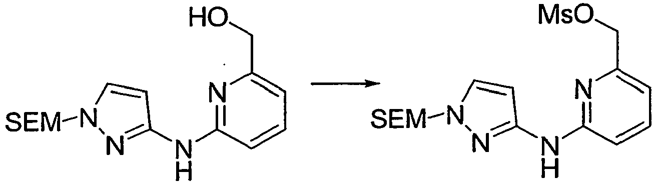

To a mixture of 10 mg of (6-((1-((2-(trimethylsilyl)ethoxy)methyl)-1H-pyrazol-3-yl)amino)pyridin-2-yl)methanol, 27 µl of N,N-diisopropylethylamine and 1 ml of chloroform was added 7.3 µl of methanesulfonyl chloride at room temperature followed by stirring for 1 hour. The reaction mixture was washed with brine, and the organic layer was dried over anhydrous magnesium sulfate and filtered. The filtrate was then concentrated *in vacuo* to give the title compound.

### (3) Synthesis of 6-((4-(3-chloro-2-fluorobenzoyl)piperazin-1-yl)methyl)-N-(1-((2-(trimethylsilyl)ethoxy)methyl)-1H-pyrazol-3-yl)pyridin-2-amine

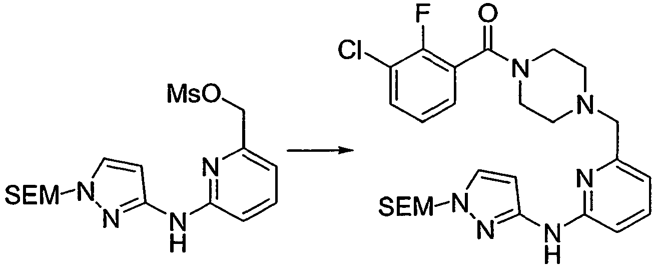

A mixture of 10 mg of 6-(methanesulfonyloxymethyl)-N-(1-((2-(trimethylsilyl)ethoxy)methyl)-1H-pyrazol-3-yl)pyridin-2-amine, 40 mg of 1-(3-chloro-2-fluorobenzoyl)piperazine hydrochlorideobtained in Reference Example 1, 27 µl of N,N-diisopropylethylamine and 1 ml of chloroform was stirred at 60°C for 5 hours. The reaction solution was diluted with chloroform, and then washed with water and brine. The resulting organic layer was dried over magnesium sulfate and filtered, and the filtrate was concentrated. The resulting residue was purified by a silica gel column chromatography(eluent: chloroform to chloroform/methanol = 20/1) to give the title compound.

### (4) Synthesis of 6-((4-(3-chloro-2-fluorobenzoyl)piperazin-1-yl)methyl)-N-1H-pyrazol-3-ylpyridin-2-amine

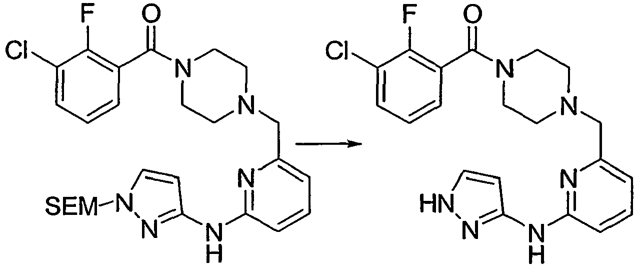

18 mg of (6-((4-(3-Chloro-2-fluorobenzoyl)piperazin-1-yl)methyl)-N-(1-((2-(trimethylsilyl)ethoxy)methyl)-1H-pyrazol-3-yl)pyridin-2-amine was dissolved in 900 µl of trifluoroacetate and 100 µl of water followed by stirring at room temperature for 5 hours. The reaction solution was concentrated *in vacuo,* diluted with ethyl acetate, and then washed with saturated sodium bicarbonate, water and brine. The resulting organic layer was dried over magnesium sulfate and filtered, and the filtrate was concentrated. The resulting residue was purified by a silica gel column chromatography (eluent: chloroform to chloroform/methanol = 10/1) to give the title compound.
Spectral data of the title compound are as follows.
¹H-NMR (CDCl₃) δ: 7.51 (dd, J = 8.2,7.4 Hz, 1H), 7.48-7.39 (m, 2H), 7.36-7.20 (m, 2H) 7.17-7.10 (m, 1H), 6.90 (d, J = 8.2 Hz, 1H), 6.82 (d, J = 7.2 Hz, 1H), 5.98 (s, 1H), 3.87 (brs, 2H), 3.62 (s, 2H), 3.37 (brs, 2H), 2.63 (t, J = 5.2 Hz, 2H), 2.51 (brs, 2H)
Mass: 415 (M + 1)⁺

Examples 6 to 9 were synthesized in the same manner as in Example 5 as follows.

### Example 6

### Synthesis of 6-((4-(2-fluoro-3-(trifluoromethyl)benzoyl)piperazin-1-yl)methyl)-N-1H-pyrazol-3-ylpyridin-2-amine trifluoroacetate

¹H-NMR (CD₃OD) δ: 8.01 (dd, J = 8.8, 7.2 Hz, 1H), 7.84 (brt, J = 7.2 Hz, 1H), 7.79 (d, J = 2.4 Hz, 1H), 7.71 (brt, J = 7.2 Hz, 1H), 7.49 (brt, J = 7.6 Hz, 1H), 7.14 (d, J = 8.8 Hz, 1H), 7.04 (dd, J = 7.2, 1.2 Hz, 1H), 6.16 (d, J = 2.4 Hz, 1H), 4.05-3.97 (m, 2H), 3.97 (s, 2H), 3.58-3.51 (m, 2H), 2.82 (brt, J = 4.8 Hz, 2H), 2.70 (brt, J = 4.8 Hz, 2H)
Mass: 449 (M + 1)⁺

### Example 7

### Synthesis of 6-((4-(3-(difluoromethyl)-2-fluorobenzoyl)piperazin-1-yl)methyl)-N-1H-pyrazol-3 - ylpyridin-2-amine

¹H-NMR (CDCl₃) δ: 7.64 (brt, J = 7.2 Hz, 1H), 7.55-7.44 (m, 3H), 7.31 (t, J = 8.0 Hz, 1H), 6.91 (d, J = 8.0 Hz, 1H), 6.89 (t, J = 54.8 Hz, 1H), 6.82 (d, J = 7.6 Hz, 1H), 5.99 (s, 1H), 3.92-3.84 (m, 2H), 3.62 (s, 2H), 3.42-3.34 (m, 2H), 2.66-2.60 (m, 2H), 2.55-2.47 (m, 2H)
Mass: 431 (M + 1)⁺

### Example 8

### Synthesis of 6-((4-(3-chloro-2-fluorobenzoyl)piperazin-1-yl)methyl)-N-(5-methyl-1H-pyrazol-3-yl)pyridin-2-amine

¹H-NMR (CD₃OD) δ: 7.99 (dd, J = 8.8, 7.2 Hz, 1H), 7.64-7.57 (m, 1H), 7.38-7.26 (m, 2H), 7.11 (d, J = 8.8 Hz, 1H), 7.01 (d, J = 7.2 Hz, 1H), 5.91 (s, 1H), 4.03-3.95 (m, 2H), 3.94 (s, 2H), 3.56-3.50 (m, 2H), 2.82-2.75 (m, 2H), 2.69-2.64 (m, 2H), 2.35 (s, 3H)
Mass: 429 (M + 1)⁺

### Example 9

### Synthesis of 6-((4-(2-fluoro-3-(trifluoromethyl)benzoyl)piperazin-1-yl)methyl)-N-(5-methyl-1H-pyrazol-3-yl)pyridin-2-amine

¹H-NMR (CD₃OD) δ: 7.90 (s, 1H), 7.81 (brt, J = 7.2 Hz, 1H), 7.68 (brt, J = 6.8 Hz, 1H), 7.53 (t, J = 8.0 Hz, 1H), 7.46 (t, J = 7.6 Hz, 1H), 6.82 (d, J = 7.2 Hz, 1H), 3.89-3.82 (m, 2H), 3.65-3.57 (m, 2H), 3.43-3.37 (m, 2H), 2.65 (brt, J = 4.8 Hz, 2H), 2.54 (brt, J = 4.8 Hz, 2H), 2.23 (s, 3H)
Mass: 463 (M + 1)⁺

### Example 10

### Synthesis of 6-((4-(2-fluoro-3-(trifluoromethyl)benzoyl)piperazin-1-yl)methyl)-N-1H-pyrazol-3-ylpyrazin-2-amine

### (1) Synthesis of 6-chloro-N-(1-((2-(trimethylsilyl)ethoxy)methyl)-1H-pyrazol-3-yl)pyrazin-2-amine

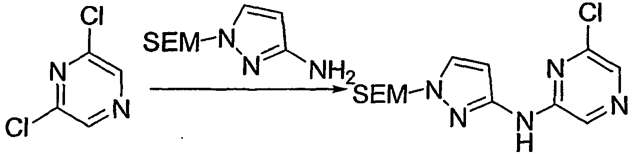

A mixture of 1.78 g of 2,6-dichloropyrazine, 2.84 g of 1-((2-(trimethylsilyl)ethoxy)methyl)-1H-pyrazol-3-amine obtained in Reference Example 2, 690 mg of 9,9-dimethyl-4,5-bis(diphenylphosphino)xanthene, 620 mg of tris(dibenzylideneacetone)dipalladium(0)-chloroform complex, 5.07 g of potassium phosphate, 25ml of 1,4-dioxane was stirred at 100°C for 2 hours, cooled to room temperature, and then diluted with ethyl acetate. An insoluble matter was filtered off using Celite and the resulting ethyl acetate solution was washed with water and brine. The organic layer was dried over anhydrous magnesium sulfate and filtered, and the filtrate was concentrated. The residue was purified by a silica gel column chromatography (eluent: hexane/ethyl acetate = 10/1 to 1/1) to give the title compound.

### (2) Synthesis of methyl 6-((1-((2-(trimethylsilyl)ethoxy)methyl)-1H-pyrazol-3-yl)amino)pyrazine-2-carboxylate

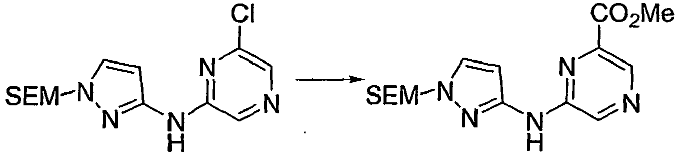

A mixture of 2.41 g of 6-chloro-N-(1-((2-(trimethylsilyl)ethoxy)methyl)-1H-pyrazol-3-yl)pyrazin-2-amine, 320 mg of palladium acetate, 790 mg of 1,1'-bisdiphenylphosphinoferrocene, 890 mg of sodium hydrogen carbonate, 10 ml of methanol and 10ml of N,N-dimethylformamide was stirred at 100°C for 15 hours under 3 atmospheric pressure of carbon monoxide, cooled to room temperature, and then diluted with ethyl acetate. An insoluble matter was filtered off using Celite and the resulting ethyl acetate solution was washed with water and brine. The organic layer was dried over anhydrous magnesium sulfate and filtered, and the filtrate was concentrated. The residue was purified by a silica gel column chromatography (eluent: hexane/ethyl acetate = 10/1 to 1/1) to give the title compound.

### (3) Synthesis of 6-((1-((2-(trimethylsilyl)ethoxy)methyl)-1H-pyrazol-3-yl)amino)pyrazine-2-carboxylic acid

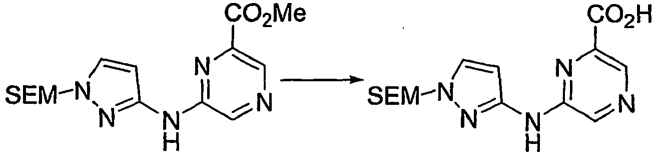

To a mixture of 52 mg of methyl 6-((1-((2-(trimethylsilyl)ethoxy)methyl)-1H-pyrazol-3-yl)amino)pyrazine-2-carboxylate, 0.5 ml of tetrahydrofuran and 1 ml of methanol was added an aqueous sodium hydroxide solution (1.0 M, 0.5 ml), followed by stirring at room temperature for 15 hours. The obtained reaction solution was diluted with ethyl acetate, and then washed with aqueous ammonium chloride and brine. The organic layer was dried over anhydrous magnesium sulfate and filtered, and filtrate was concentrated to give the title compound.

### (4) Synthesis of (6-((1-((2-(trimethylsilyl)ethoxy)methyl)-1H-pyrazol-3-yl)amino)pyrazin-2-yl)methanol

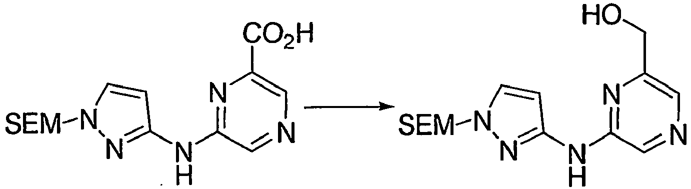

To a mixture of 28 mg of 6-((1-((2-(trimethylsilyl)ethoxy)methyl)-1H-pyrazol-3-yl)amino)pyrazine-2-carboxylic acid and 1 ml of N,N-dimethylformamide was added 84 mg of N,N'-carbonyldiimidazole, followed by stirring at room temperature for 15 hours. Then, 200 µl of an aqueous solution of 20 mg of sodium borohydride was added thereto and the resulting mixture was stirred. Water was added to the reaction mixture, and then the mixture was extracted with ethyl acetate. The resulting ethyl acetate solution was dried over anhydrous magnesium sulfate and filtered, and the filtrate was concentrated. The residue was purified by a silica gel column chromatography (eluent: chloroform to chloroform/methanol = 10/1) to give the title compound.

### (5) Synthesis of 6-((4-(2-fluoro-3-(trifluoromethyl)benzoyl)piperazin-1-yl)methyl)-N-(1-((2-(trimethylsilyl)ethoxy)methyl)-1H-pyrazol-3-yl)pyrazin-2-amine

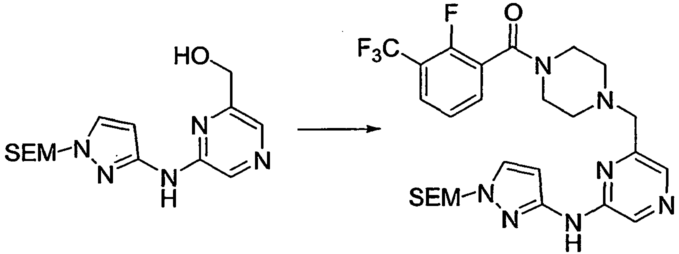

To a mixture of 2.14 g of (6-((1-((2-(trimethylsilyl)ethoxy)methyl)-1H-pyrazol-3-yl)amino)pyrazin-2-yl)methanol, 2.32 ml of N,N-diisopropylethylamine and 40 ml of chloroform was added 619 µl of methanesulfonyl chloride at room temperature followed by stirring for 1 hour. To the reaction mixture was added 2.32 ml of N,N-diisopropylethylamine, and then 3.13 g of 1-(2-fluoro-3-(trifluoromethyl)benzoyl)piperazine hydrochloride obtained in Reference Example 3 was added thereto followed by stirring at 50°C for 2 hours. The resulting reaction mixture was washed with aqueous sodium bicarbonate and brine. The resulting organic layer was dried over anhydrous magnesium sulfate and filtered, and then the filtrate was concentrated *in vacuo.* The resulting residue was purified by a silica gel column chromatography (eluent: chloroform to chloroform/methanol = 20/1) to obtain the title compound.

### (6) Synthesis of 6-((4-(2-fluoro-3-(trifluoromethyl)benzoyl)piperazin-1-yl)methyl)-N-1H-pyrazol-3-ylpyrazin-2-amine

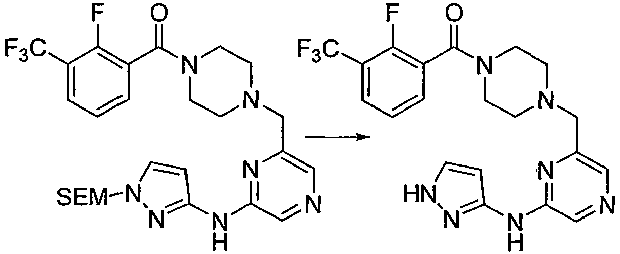

2.49 g of 6-((4-(2-fluoro-3-(trifluoromethyl)benzoyl)piperazin-1-yl)methyl)-N-(1-((2-(trimethylsilyl)ethoxy)methyl)-1H-pyrazol-3-yl)pyrazin-2-amine was dissolved in 25 ml of trifluoroacetic acid and 2.5 ml of water followed by stirring at room temperature for 2 hours. The reaction solution was concentrated *in vacuo,* diluted with ethyl acetate, and then washed with saturated sodium bicarbonate, water and brine. The resulting organic layer was dried over magnesium sulfate and filtered, and the filtrate was concentrated. The resulting residue was purified by a silica gel column chromatography (eluent: chloroform to chloroform/methanol = 5/1) to give the title compound.
Spectral data of the title compound are as follows.
¹H-NMR (CDCl₃) δ: 8.51 (s, 1H), 8.09 (s, 1H), 7.70-7.56 (m, 2H), 7.52 (s, 1H), 7.37-7.20 (m, 2H), 6.26 (s, 1H), 3.89 (brs, 2H), 3.64 (s, 2H), 3.37 (brs, 2H), 2.66 (dd, J = 5.1,4.9 Hz, 2H), 2.54 (brs, 2H)
Mass: 450 (M + 1)⁺

Examples 11 to 15 were synthesized in the same manner as in Example 10 as follows.

### Example 11

### Synthesis of 6-(((1S,4S)-5-(3-chloro-2-fluorobenzoyl)-2,5-diazabicyclo[2.2.12]hept-2-yl)methyl)-N-1H-pyrazol-3-ylpylazin-2-amine

¹H-NMR (CDCl₃) δ: 8.44 (s, 1H), 8.11-8.03 (m, 1H), 7.87-7.65 (m, 1H), 7.57-7.42 (m, 2H), 7.40-7.30 (m, 1H), 7.22-7.13 (m, 1H), 6.34-6.22 (m, 1H), 4.97-2.70 (m, 8H), 2.10-1.80 (m, 2H)
Mass: 428 (M + 1)⁺

### Example 12

### Synthesis of 6-((4-(3-chloro-2-fluorobenzoyl)piperazin-1-yl)methyl)-N-1H-pyrazol-3-ylpyrazin-2-amine

¹H-NMR (CDCl₃) δ: 8.51 (s, 1H), 8.09 (s, 1H), 7.52 (s, 1H), 7.45 (dd, J = 7.0, 6.3 Hz, 1H), 7.37-7.24 (m, 2H), 7.15 (t, J = 7.8 Hz, 1H), 6.26 (s, 1H), 3.86 (brs, 2H), 3.64 (s, 2H), 3.38 (brs, 2H), 2.65 (dd, J = 5.3, 4.7 Hz, 2H), 2.53 (brs, 2H)

### Example 13

### Synthesis of 6-((4-(3-chloro-2-fluorobenzoyl)piperazin-1-yl)methyl)-N-(5-methyl-1H-pyrazol-3-yl)pyrazin-2-amine

¹H-NMR (CDCl₃) δ: 8.48 (s, 1H), 8.06 (s, 1H), 7.48-7.33 (m, 2H), 7.33-7.25 (m, 1H), 7.15 (dd, J = 8.0,7.6 Hz, 1H), 6.02 (s, 1H), 3.87 (brs, 2H), 3.62 (s, 2H), 3.37 (brs, 2H), 2.64 (t, J = 5.1 Hz, 2H), 2.52 (brs, 2H), 2.31 (s, 3H)
Mass: 430 (M + 1)⁺

### Example 14

### Synthesis of 6-((4-(3-chloro-2-fluorobenzoyl)piperazin-1-yl)methyl)-N-thiazol-2-ylpyrazin-2-amine

¹H-NMR (CDCl₃) δ: 8.33 (s, 1H), 8.24 (s, 1H), 7.56 (d, J = 3.5 Hz, 1H), 7.49-7.42 (m, 1H), 7.30-7.23 (m, 2H), 7.14 (t, J = 8.1 Hz, 1H), 6.95 (d, J = 3.7 Hz, 1H), 3.87 (brs, 2H), 3.79 (s, 2H), 3.34 (brs, 2H), 2.72 (t, J = 5.2 Hz, 2H), 2.59 (brs, 2H)
Mass: 433 (M + 1)⁺

### Example 15

### Synthesis of 6-((4-(3-chloro-2-fluorobenzoyl)piperazin-1-yl)methyl)-N-(5-methyl-1H-pyrazol-3-yl)pyrazin-2-amine

¹H-NMR (CDCl₃) δ: 8.48 (s, 1H), 8.06 (s, 1H), 7.67 (t, *J* = 7.3 Hz, 1H), 7.60 (t, *J* = 6.6 Hz, 1H), 7.33 (t, *J* = 7.6 Hz, 1H), 7.18 (s, 1H), 6.03 (s, 1H), 3.90-3.80 (brs, 2H), 3.63 (s, 2H), 3.50-3.30 (brs, 2H), 2.80-2.45 (m, 4H), 2.33 (s, 3H)
Mass: 464 (M + 1)⁺

### Example 16

### Synthesis of 6-((4-(3-chloro-2-fluorobenzoyl)piperazin-1-yl)methyl)-N-(5-chlorothiazol-2-yl)pyridin-2-amine

### (1) Synthesis of 6-(((tert-butyl(dimethyl)silyl)oxy)methyl)-N-(5-chlorothiazol-2-yl)pyridin-2-amine

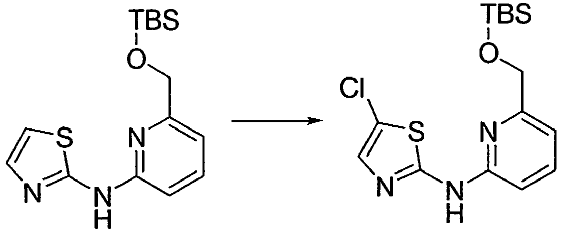

2 g of 6-(((tert-butyl(dimethyl)silyl)oxy)methyl)-N-thiazol-2-ylpyridin-2-amine obtained in Example 1-(2) was dissolved in 20 ml of 1,4-dioxane, and then 832 mg of N-chlorosuccinimide was added thereto at room temperature. The reaction mixture was heated under reflux for 2 hours, cooled to room temperature, diluted with ethyl acetate, and then washed with water and brine. The resulting organic layer was dried over anhydrous magnesium sulfate and filtered, and the filtrate was concentrated *in vacuo* to give the title compound.

### (2) Synthesis of 6-(chloromethyl)-N-(5-chlorothiazol-2-yl)pyridin-2-amine

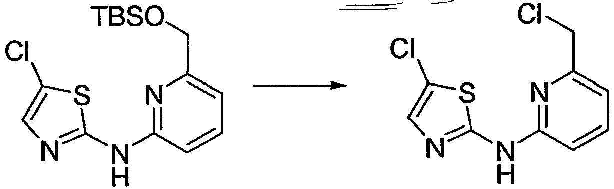

In the same manner as in Example 1-(3) and (4), the title compound was obtained using 6-(((tert-butyl(dimethyl)silyl)oxy)methyl)- N-(5-chlorothiazol-2-yl)pyridin-2-amine.

### (3) Synthesis of 6-((4-(3-chloro-2-fluorobenzoyl)piperazin-1-yl)methyl)-N-(5-chlorothiazol-2-yl)pyridin-2-amine

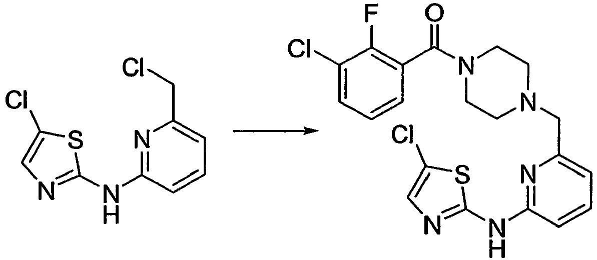

In the same manner as in Example 1-(5), the title compound was obtained using 6-(chloromethyl)-N-(5-chlorothiazol-2-yl)pyridin-2-amine. Spectral data of the title compound are as follows.
¹H-NMR (DMSO-d₆) δ: 11.46 (s, 1H), 7.73-7.62 (m, 2H), 7.41-7.33 (m, 2H), 7.29 (t, J = 8.0 Hz, 1H), 7.01 (d, J = 7.4 Hz, 1H), 6.89 (d, J = 8.2 Hz, 1H), 3.73-3.62 (m, 4H), 3.36-3.20 (m, 2H), 2.59-2.39 (m, 4H)
Mass: 466 (M + 1)⁺

### [Reference Example]

### Reference Example 1

### Synthesis of 1-(3-chloro-2-fluorobenzoyl)piperazine hydrochloride

### (1) Synthesis of tert-butyl 4-(3-chloro-2-fluorobenzoyl)piperazine-1-carboxylate

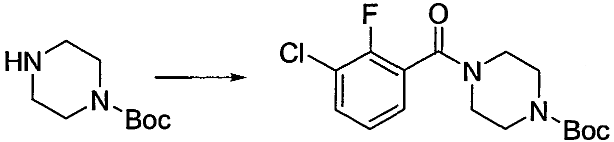

A mixture of 19.4 g of 1-Boc-piperazine, 24.0 g of 1-(3-dimethylaminopropyl)-3-ethylcarbodiimide hydrochloride, 19.1 g of 1-hydroxybenzotriazole, 20.0 g of 3-chloro-2-fluorobenzoic acid and 200 ml of chloroform was stirred at room temperature for 4 hours. The resulting mixture was diluted with chloroform, and an insoluble matter was filtered off using Celite. Then, the filtrate was washed with water and brine. The resulting organic layer was dried over magnesium sulfate and filtered, and the filtrate was concentrated *in vacuo.* The resulting residue was purified by a silica gel column chromatography (eluent: hexane/ethyl acetate = 9/1 to 1/1) to give title compound.

### (2) Synthesis of 1-(3-chloro-2-fluorobenzoyl)piperazine hydrochloride

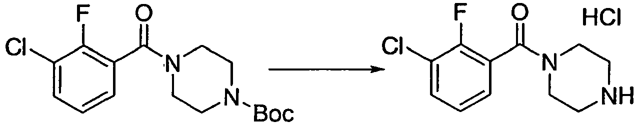

To a mixture of 35.1 g of tert-butyl 4-(3-chloro-2-fluorobenzoyl)piperazine-1-carboxylate and 50 ml of methanol was added a hydrochloric acid-1,4-dioxane solution (4 M, 100 ml) followed by stirring at room temperature for 2.5 hours. The reaction mixture was concentrated *in vacuo* and the residue was suspended in diethyl ether, filtered and collected to give the title compound.
Spectral data of the title compound are as follows.
¹H-NMR (DMSO-d₆) δ: 9.68 (br ,1 H) ,7.74-7.65s (m, 1H), 7.50-7.41 (m, 1H), 7.37-7.28 (m, 1H), 3.88 (br, 2H), 3.57-3.30 (m, 2H), 3.16 (br, 2H), 3.03 (br, 2H)
Mass: 243 (M + 1)⁺

### Reference Example 2

### Synthesis of 1-((2-(trimethylsilyl)ethoxy)methyl)-1H-pyrazol-3-amine

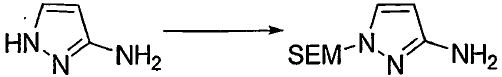

To a solution of 10 g of 1H-pyrazol-3-amine in 100 ml of N,N-dimethylformamide was added 9.6 g of sodium hydride (60%, in oil) under cooling with ice. The reaction mixture was stirred for 30 minutes, and then 21.3 ml of 2-(trimethylsilyl)ethoxymethyl chloride was added thereto. After stirring the resulting mixture at room temperature for 1 hour, aqueous ammonium chloride was added thereto, and the mixture was extracted with chloroform. The resulting organic layer was washed with water and brine, and then dried over magnesium sulfate. The organic layer was filtered and concentrated *in vacuo.* The resulting residue was purified by a silica gel column chromatography (eluent: hexane/ethyl acetate = 4/1 to 1/2) to give the title compound.

### Reference Example 3

### Synthesis of 1-(2-fluoro-3-(trifluoromethyl)benzoyl)piperazine hydrochloride

### (1) Synthesis of tert-butyl 4-(2-fluoro-3-(trifluoromethyl)benzoyl)piperazine-1-carboxylate

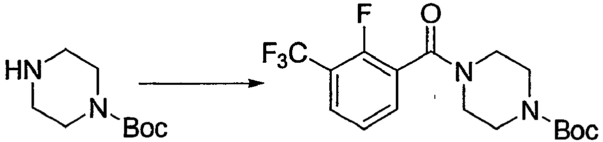

A mixture of 3.66 g of 1-Boc-piperazine, 4.53 g of 1-(3-dimethylaminopropyl)-3-ethylcarbodiimide hydrochloride, 3.61 g of 1-hydroxybenzotriazole, 4.50 g of 2-fluoro-3-(trifluoromethyl)benzoic acid and 40 ml of chloroform was stirred at room temperature for 4 hours. The reaction mixture was diluted with chloroform and then was washed with water and brine. The resulting organic layer was dried over magnesium sulfate and filtered, and the filtrate was concentrated *in vacuo.* The resulting residue was purified by a silica gel column chromatography (eluent: hexane/ethyl acetate = 4/1 to 2/1) to give the title compound.

### (2) Synthesis of 1-(2-fluoro-3-(trifluoromethyl)benzoyl)piperazine hydrochloride

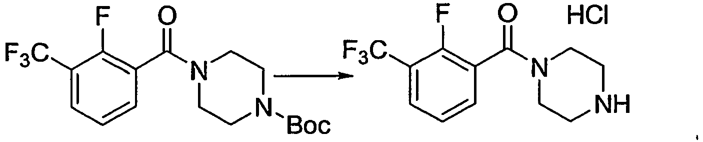

To a mixture of 7.74 g of tert-butyl 4-(2-fluoro-3-(trifluoromethyl)benzoyl)piperazine-1-carboxylate and 10 ml of methanol was added a hydrochloric acid-1,4-dioxane solution (4 M, 20 ml) followed by stirring at room temperature for 4 hours. The reaction mixture was concentrated *in vacuo* to give the title compound.
Spectral data of the title compound are as follows.
¹H-NMR (DMSO-d₆) δ: 7.95-7.81 (m, 2H), 7.56-7.49 (m, 1H), 3.92-3.79 (m, 2H), 3.45 (br, 2H), 3.19 (br, 2H), 3.04 (br, 2H)
Mass: 277 (M + 1)

### Industrial Applicability

The compound of the invention is characterized in that it has cell growth inhibitory action as well as synergistic action with other antitumor agents, based on excellent Aurora A selective inhibitory action, and thus it is expected as a useful antitumor agent in the field of pharmaceuticals.

## Claims

1. A compound of general formula I: 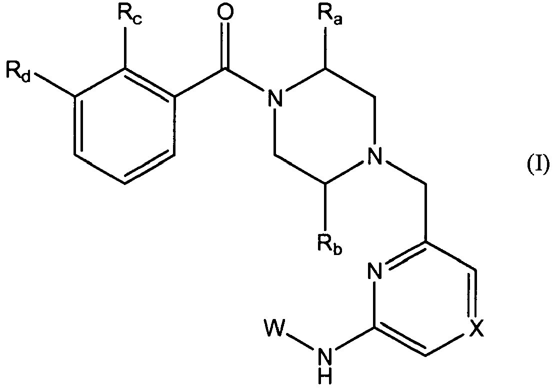 wherein:
Rₐ and R_{b} are each independently hydrogen atom or a linear or branched alkyl group having 1 to 6 carbon atoms, or alternatively Rₐ and R_{b} are combined together to form -CH₂-;
R_{c} is halogen atom;
R_{d} is halogen atom or methyl substituted with one to three halogen atoms;
X is CH or N; and
W is selected from: 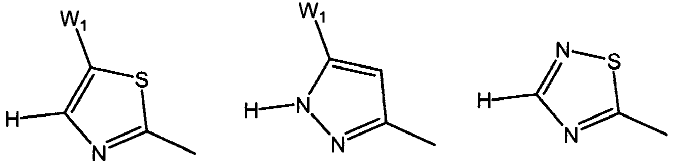
wherein W₁ is hydrogen atom, halogen atom, or methyl which may be substituted with one to three fluorine atoms;
with the proviso that when W is 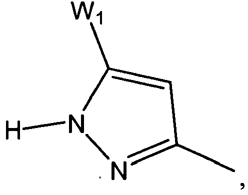 then X is N;
or a pharmaceutically acceptable salt thereof.

2. A compound of claim 1 which is:
(a) 6-((4-(3-chloro-2-fluorobenzoyl)piperazin-1-yl)methyl)-N-thiazol-2-ylpyridin-2-amine;
(b) 6-((4-(2,3-dichlorobenzoyl)piperazin-1-yl)methyl)-N-thiazol-2-ylpyridin-2-amine;
(c) 6-(((1S,4S)-5-(3-chloro-2-fluorobenzoyl)-2,5-diazabicyclo(2.2.1)hept-2-yl)methyl)-N-thiazol-2-ylpyridin-2-amine;
(d) 6-((4-(3-chloro-2-fluorobenzoyl)piperazin-1-yl)methyl)-N-1H-pyrazol-3-ylpyridin-2-amine;
(e) 6-((4-(2-fluoro-3-(trifluoromethyl)benzoyl)piperazin-1-yl)methyl)-N-1H-pyrazol-3-ylpyridin-2-amine;
(f) 6-((4-(3-(difluoromethyl)-2-fluorobenzoyl)piperazin-1-yl)methyl)-N-1H-pyrazol-3-ylpyridin-2-amine;
(g) 6-((4-(3-chloro-2-fluorobenzoyl)piperazin-1-yl)methyl)-N-(5-methyl-1H-pyrazol-3-yl)pyridin-2-amine;
(h) 6-((4-(2-fluoro-3-(trifluoromethyl)benzoyl)piperazin-1-yl)methyl)-N-(5-methyl-1H-pyrazol-3- yl)pyridin-2-amine;
(i) 6-((4-(2-fluoro-3-(trifluoromethyl)benzoyl)piperazin-1-yl)methyl)-N-1,2,4-thiadiazol-5-ylpyridin-2-amine;
(j) 6-((4-(2-fluoro-3-(trifluoromethyl)benzoyl)piperazin-1-yl)methyl)-N-1H-pyrazol-3-ylpyrazin-2- amine;
(k) 6-(((1S,4S)-5-(3-chloro-2-fluorobenzoyl)-2,5-diazabicyclo(2.2.1)hept-2-yl)methyl)-N-1H-pyrazol-3-ylpyrazin-2-amine;
(l) 6-((4-(2-fluoro-3-(trifluoromethyl)benzoyl)piperazin-1-yl)methyl)-N-1H-pyrazol-3-ylpyrazin-2- amine;
(m) 6-((4-(3-chloro-2-fluorobenzoyl)piperazin-1-yl)methyl)-N-(5-methyl-1H-pyrazol-3-yl)pyrazin-2-amine;
(n) 6-((4-(3-chloro-2-fluorobenzoyl)piperazin-1-yl)methyl)-N-thiazol-2-ylpyrazin-2-amine;
(o) 6-((4-(3-chloro-2-fluorobenzoyl)piperazin-1-yl)methyl)-N-(5-chlorothiazol-2-yl)pyridin-2-amine; or
(p) 6-((4-(2-fluorobenzoyl-3-trifluoromethyl)piperazin-1-yl)methyl)-N-(5-methyl-1H-pyrazol-3-yl) pyrazin-2-amine;
or a pharmaceutically acceptable salt thereof.

3. The compound according to claim 2 which is:
6-((4-(3-chloro-2-fluorobenzoyl)piperazin-1-yl)methyl)-N-thiazol-2-ylpyridin-2-amine;
or a pharmaceutically acceptable salt thereof.

4. The compound according to claims 2 which is:
6-((4-(2-fluoro-3-(trifluoromethyl)benzoyl)piperazin-1-yl)methyl)-N-1H-pyrazol-3-ylpyridin-2-amine;
or a pharmaceutically acceptable salt thereof.

5. The compound according to claim 2 which is:
6-((4-(2-fluoro-3-(trifluoromethyl)benzoyl)piperazin-1-yl)methyl)-N-1H-pyrazol-3-ylpyrazin-2-amine;
or a pharmaceutically acceptable salt thereof.

6. The compound according to claim 2 which is:
6-(((1S,4S)-5-(3-chloro-2-fluorobenzoyl)-2,5-diazabicyclo(2.2.1)hept-2-yl)methyl)-N-1H-pyrazol-3-ylpyrazin-2-amine;
or a pharmaceutically acceptable salt thereof.

7. A pharmaceutical composition comprising a compound of any previous claim, or a pharmaceutically acceptable salt thereof together with a pharmaceutically acceptable carrier or diluent.

8. A compound of any one of claims 1 to 6, or a pharmaceutically acceptable salt thereof for use in therapy.

9. A combination of a compound of any one of claims 1 to 6, or a pharmaceutically acceptable salt thereof and at least one other antitumor agent for simultaneous, separate or sequential administration.

10. A combination of
*a preparation comprising, together with a pharmaceutically acceptable carrier or diluent, a compound according to any one of claims 1 to 6; and
*a preparation comprising, together with a pharmaceutically acceptable carrier or diluent, one antitumor agent selected from the group consisting of antitumor alkylating agents, antitumor antimetabolites, antitumor antibiotics, plant-derived antitumor agents, antitumor platinum-complex compounds, antitumor campthotecin derivatives, antitumor tyrosine kinase inhibitors, monoclonal antibodies, interferons, biological response modifiers, and other antitumor agents or a pharmaceutically acceptable salt thereof, wherein:
the antitumor alkylating agents are nitrogen mustard N-oxide, cyclophosphamide, ifosfamide, melphalan, busulfan, mitobronitol, carboquone, thiotepa, ranimustine, nimustine, temozolomide, and camustine;
the antitumor antimetabolites arc methotrexate, 6-mercaptopurine riboside, mercaptopurine, 5-fluorouracil, tegafur, doxifluridine, carmofur, cytarabine, cytarabine ocfosfate, enocitabine, S-1, gemcitabine, fludarabine, and pemetrexed disodium;
the antitumor antibiotics are actinomycin D, doxorubicin, daunorubicin, neocarzinostatin, bleomycin, peplomycin, mitomycin C, aclarubicin, pirarubicin, epirubicin, zinostatin stimalamer, idarubicin, sirolimus, and valrubicin;
the plant-derived antitumor agents are vincristine, vinblastine, vindeshine, etoposide, sobuzoxane, docetaxel, paclitaxel, and vinorelbine;
the antitumor platinum-complex compounds are cisplatin, carboplatin, nedaplatin, and oxaliplatin;
the antitumor campthotecin derivatives are irinotecan, topotecan, and campthotecin;
the antitumor tyrosine kinase inhibitors are gefitinib, imatinib, and erlotinib;
the monoclonal antibodies are cetuximab, bevacizumab, rituximab, bevacizumab, alemtuzumab, and trastuzumab;
the interferons are interferon α, interferon α-2a, interferon α-2b, interferon β, interferon γ-1a, and interferon γ-n1;
the biological response modifiers are krestin, lentinan, sizofiran, picibanil, or ubenimex; and
the other antitumor agents are mitoxantrone, L-asparaginase, procarbazine, dacarbazine, hydroxycarbamide, pentostatin, tretinoin, alefacept, darbepoetin alfa, anastrozole, exemestane, bicalutamide, leuprorelin, flutamide, fulvestrant, pegaptanib octasodium, denileukin diftitox, aldesleukin, thyrotropin alfa, arsenic trioxide, bortezomib, capecitabine, and goserelin, for simultaneous, separate or sequential administration.

11. A combination of claim 10 wherein the antitumor agent is paclitaxel or docetaxyl.

12. The use of a compound of any one of claims 1 to 6, or a pharmaceutically acceptable salt thereof for the manufacture of a medicament for treating or preventing cancer.

13. The use of a combination of any one of claims 9 to 11 for the manufacture of a medicament for treating or preventing cancer.

14. A compound of any one of claims 1 to 6, or a pharmaceutically acceptable salt thereof, for use in a method of treating or preventing cancer.

## Patentansprüche

1. Eine Verbindung der allgemeinen Formel I: 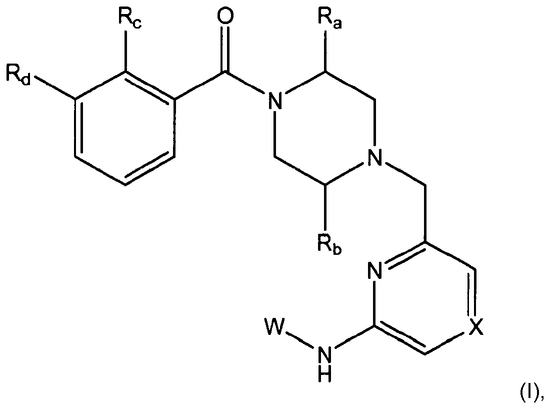 wobei:
Rₐ und R_{b} jeweils unabhängig ein Wasserstoffatom oder eine lineare oder verzweigte Alkylgruppe mit 1 bis 6 Kohlenstoffatomen sind, oder, alternativ, Rₐ und R_{b} miteinander verbunden sind unter Bildung von -CH₂-,
R_{c} ein Halogenatom ist,
R_{d} ein Halogenatom oder ein mit einem bis drei Halogenatomen substituiertes Methyl ist,
X CH oder N ist und
W ausgewählt ist aus: 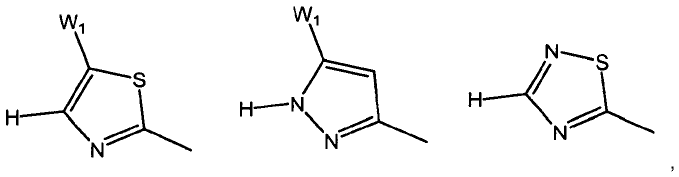
wobei W₁ ein Wasserstoffatom, ein Halogenatom oder Methyl, das mit einem bis drei Fluoratomen substituiert sein kann, ist,
mit der Maßgabe, dass, wenn W 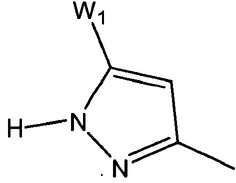 ist, X dann N ist, oder ein pharmazeutisch annehmbares Salz davon.

2. Eine Verbindung nach Anspruch 1, die ist:
(a) 6-((4-(3-Chlor-2-fluorbenzoyl)piperazin-1-yl)methyl)-N-thiazol-2-ylpyridin-2-amin,
(b) 6-((4-(2,3-Dichlorbenzoyl)piperazin-1-yl)methyl)-N-thiazol-2-ylpyridin-2-amin,
(c) 6-(((1S,4S)-5-(3-Chlor-2-fluorbenzoyl)-2,5-diazabicyclo(2.2.1)hept-2-yl)methyl)-N-thiazol-2-ylpyridin-2-amin,
(d) 6-((4-(3-Chlor-2-fluorbenzoyl)piperazin-1-yl)methyl)-N-1H-pyrazol-3-ylpyridin-2-amin,
(e) 6-((4-(2-Fluor-3-(trifluormethyl)benzoyl)piperazin-1-yl)methyl)-N-1H-pyrazol-3-ylpyridin-2-amin,
(f) 6-((4-(3-(Difluormethyl)-2-fluorbenzoyl)piperazin-1-yl)methyl)-N-1H-pyrazol-3-ylpyridin-2-amin,
(g) 6-((4-(3-Chlor-2-fluorbenzoyl)piperazin-1-yl)methyl)-N-(5-methyl-1H-pyrazol-3-yl)pyridin-2-amin,
(h) 6-((4-(2-Fluor-3-(trifluormethyl)benzoyl)piperazin-1-yl)methyl)-N-(5-methyl-1H-pyrazol-3-yl)pyridin-2-amin,
(i) 6-((4-(2-Fluor-3-(trifluormethyl)benzoyl)piperazin-1-yl)methyl)-N-1,2,4-thiadiazol-5-ylpyridin-2-amin,
(j) 6-((4-(2-Fluor-3-(trifluormethyl)benzoyl)piperazin-1-yl)methyl)-N-1H-pyrazol-3-ylpyrazin-2-amin,
(k) 6-(((1S,4S)-5-(3-Chlor-2-fluorbenzoyl)-2,5-diazabicyclo(2.2.1)hept-2-yl)methyl)-N-1H-pyrazol-3-ylpyrazin-2-amin,
(l) 6-((4-(2-Fluor-3-(trifluormethyl)benzoyl)piperazin-1-yl)methyl)-N-1H-pyrazol-3-ylpyrazin-2-amin,
(m) 6-((4-(3-Chlor-2-fluorbenzoyl)piperazin-1-yl)methyl)-N-(5-methyl-1H-pyrazol-3-yl)pyrazin-2-amin,
(n) 6-((4-(3-Chlor-2-fluorbenzoyl)piperazin-1-yl)methyl)-N-thiazol-2-ylpyrazin-2-amin,
(o) 6-((4-(3-Chlor-2-fluorbenzoyl)piperazin-1-yl)methyl)-N-(5-chlorthiazol-2-yl)pyridin-2-amin oder
(p) 6-((4-(2-Fluorbenzoyl-3-trifluormethyl)piperazin-1-yl)methyl)-N-(5-methyl-1H-pyrazol-3-yl)pyrazin-2-amin,
oder ein pharmazeutisch annehmbares Salz davon.

3. Die Verbindung gemäß Anspruch 2, die ist:
6-((4-(3-Chlor-2-fluorbenzoyl)piperazin-1-yl)methyl)-N-thiazol-2-ylpyridin-2-amin,
oder ein pharmazeutisch annehmbares Salz davon.

4. Die Verbindung gemäß Anspruch 2, die ist:
6-((4-(2-Fluor-3-(trifluormethyl)benzoyl)piperazin-1-yl)methyl)-N-1H-pyrazol-3-ylpyridin-2-amin,
oder ein pharmazeutisch annehmbares Salz davon.

5. Die Verbindung gemäß Anspruch 2, die ist:
6-((4-(2-Fluor-3-(trifluormethyl)benzoyl)piperazin-1-yl)methyl)-N-1H-pyrazol-3-ylpyrazin-2-amin,
oder ein pharmazeutisch annehmbares Salz davon.

6. Die Verbindung gemäß Anspruch 2, die ist:
6-(((1S,4S)-5-(3-Chlor-2-fluorbenzoyl)-2,5-diazabicyclo(2.2.1)hept-2-yl)methyl)-N-1H-pyrazol-3-ylpyrazin-2-amin,
oder ein pharmazeutisch annehmbares Salz davon.

7. Eine pharmazeutische Zusammensetzung, umfassend eine Verbindung nach einem vorhergehenden Anspruch oder ein pharmazeutisch annehmbares Salz davon zusammen mit einem pharmazeutisch annehmbaren Träger oder Verdünnungsmittel.

8. Eine Verbindung nach einem der Ansprüche 1 bis 6 oder ein pharmazeutisch annehmbares Salz davon zur Verwendung in der Therapie.

9. Eine Kombination aus einer Verbindung nach einem der Ansprüche 1 bis 6 oder ein pharmazeutisch annehmbares Salz davon und wenigstens einem anderen Antitumormittel zur gleichzeitigen, separaten oder sequentiellen Verabreichung.

10. Eine Kombination aus
* einem Präparat, umfassend, zusammen mit einem pharmazeutisch annehmbaren Träger oder Verdünnungsmittel, eine Verbindung gemäß einem der Ansprüche 1 bis 6, und
* einem Präparat, umfassend, zusammen mit einem pharmazeutisch annehmbaren Träger oder Verdünnungsmittel, ein Antitumormittel, ausgewählt aus der Gruppe, bestehend aus antitumoralen Alkylierungsmitteln, antitumoralen Antimetaboliten, antitumoralen Antibiotika, Antitumormitteln pflanzlichen Ursprungs, antitumoralen Platinkomplexverbindungen, antitumoralen Campthotecin-Derivaten, antitumoralen Tyrosinkinaseinhibitoren, monoklonalen Antikörpern, Interferonen, Biomodulatoren und anderen Antitumormitteln, oder einem pharmazeutisch annehmbaren Salz davon, wobei:
die antitumoralen Alkylierungsmittel Stickstofflost-N-oxid, Cyclophosphamid, Ifosfamid, Melphalan, Busulfan, Mitobronitol, Carboquon, Thiotepa, Ranimustin, Nimustin, Temozolomid und Carmustin sind,
die antitumoralen Antimetabolite Methotrexat, 6-Mercaptopurinribosid, Mercaptopurin, 5-Fluorouracil, Tegafur, Doxifluridin, Carmofur, Cytarabin, Cytarabinocfosfat, Enocitabin, S-1, Gemcitabin, Fludarabin und Pemetrexed-Dinatrium sind,
die antitumoralen Antibiotika Actinomycin D, Doxorubicin, Daunorubicin, Neocarzinostatin, Bleomycin, Peplomycin, Mitomycin C, Aclarubicin, Pirarubicin, Epirubicin, Zinostatin-Stimalamer, Idarubicin, Sirolimus und Valrubicin sind,
die Antitumormittel pflanzlichen Ursprungs Vincristin, Vinblastin, Vindeshin, Etoposid, Sobuzoxan, Docetaxel, Paclitaxel und Vinorelbin sind,
die antitumoralen Platinkomplexverbindungen Cisplatin, Carboplatin, Nedaplatin und Oxaliplatin sind,
die antitumoralen Campthotecin-Derivate Irinotecan, Topotecan undr Campthotecin sind,
die antitumoralen Tyrosinkinaseinhibitoren Gefitinib, Imatinib und Erlotinib sind,
die monoklonalen Antikörper Cetuximab, Bevacizumab, Rituximab, Bevacizumab, Alemtuzumab und Trastuzumab sind,
die Interferone Interferon α, Interferon α-2a, Interferon α-2b, Interferon β, Interferon-γ-1a und Interferon γ-n1 sind,
die Biomodulatoren Krestin, Lentinan, Sizofiran, Picibanil oder Ubenimex sind und
die anderen Antitumormittel Mitoxantron, L-Asparaginase, Procarbazin, Dacarbazin, Hydroxycarbamid, Pentostatin, Tretinoin, Alefacept, Darbepoetin-alfa, Anastrozol, Exemestan, Bicalutamid, Leuprorelin, Flutamid, Fulvestrant, Pegaptanib-Octanatrium, Denileukin-Diftitox, Aldesleukin, Thyrotropin-alfa, Arsentrioxid, Bortezomib, Capecitabin oder Goserelin sind, zur gleichzeitigen, separaten oder sequentiellen Verabreichung.

11. Eine Kombination nach Anspruch 10, wobei das Antitumormittel Paclitaxel oder Docetaxyl ist.

12. Die Verwendung einer Verbindung nach einem der Ansprüche 1 bis 6 oder eines pharmazeutisch annehmbaren Salzes davon zur Herstellung eines Medikaments zur Behandlung oder Prävention von Krebs.

13. Die Verwendung einer Kombination nach einem der Ansprüche 9 bis 11 zur Herstellung eines Medikaments zur Behandlung oder Prävention von Krebs.

14. Eine Verbindung nach einem der Ansprüche 1 bis 6 oder ein pharmazeutisch annehmbares Salz davon zur Verwendung bei einem Verfahren zur Behandlung oder Prävention von Krebs.

## Revendications

1. Composé de la formule générale I: 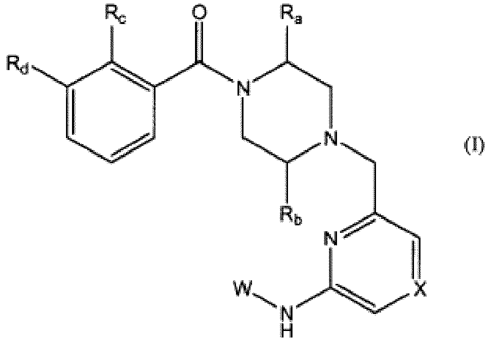 où:
R^{a} et R^{b} sont chacun indépendamment un atome d'hydrogène ou un groupe alkyle linéaire ou ramifié ayant 1 à 6 atomes de carbone ou bien alternativement Rₐ et R_{b} sont combinés ensemble pour former -CH₂-;
R_{c} est un atome d'halogène;
R_{d} est un atome d'halogène ou un méthyle substitué par un à trois atomes d'halogène;
X est CH ou N; et
W est sélectionné parmi: 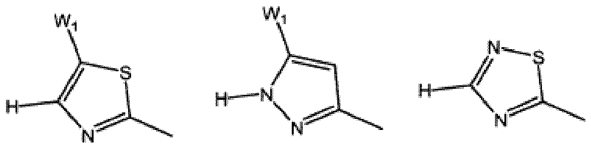
où W₁ est un atome d'hydrogène, un atome d'halogène ou un méthyle qui peut être substitué par un à trois atomes de fluor;
à condition que lorsque W est 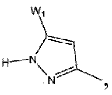 alors X est N;
ou un sel pharmaceutiquement acceptable de celui-ci.

2. Composé selon la revendication 1, qui est de la:
(a) 6-((4-(3-chloro-2-fluorobenzoyl)pipérazin-1-yl)méthyl)-N-thiazol-2-ylpyridin-2-amine;
(b) 6-((4-(2,3-dichlorobenzoyl)pipérazin-1-yl)méthyl)-N-thiazol-2-ylpyridin-2-amine;
(c) 6-(((1S,4S)-5-(3-chloro-2-fluorobenzoyl)-2,5-diazabicyclo(2.2.1)hept-2-yl)méthyl)-N-thiazol-2-ylpyridin-2-amine;
(d) 6-((4-(3-chloro-2-fluorobenzoyl)pipérazin-1-yl)méthyl)-N-1H-pyrazol-3-ylpyridin-2-amine;
(e) 6-((4-(2-fluoro-3-(trifluorométhyl)benzoyl)pipérazin-1-yl)méthyl)-N-1H-pyrazol-3-ylpyridin-2-amine;
(f) 6-((4-(3-(trifluorométhyl)-2-fluorobenzoyl)pipérazin-1-yl)méthyl)-N-1H-pyrazol-3-ylpyridin-2-amine;
(g) 6-((4-(3-chloro-2-fluorobenzoyl)pipérazin-1-yl)méthyl)-N-(5-méthyl-1H-pyrazol-3-yl)pyridin-2-amine;
(h) 6-((4-(2-fluoro-3-(trifluorométhyl)benzoyl)pipérazin-1-yl)méthyl)-N-(5-méthyl-1H-pyrazol-3-yl)pyridin-2-amine,
(i) 6-((4-(2-fluoro-3-(trifluorométhyl)benzoyl)pipérazin-1-yl)méthyl)-N-1,2,4-thiadiazol-5-ylpyridin-2-amine;
(j) 6-((4-(2-fluoro-3-(trifluorométhyl)benzoyl)pipérazin-1-yl)méthyl)-N-1H-pyrazol-3-ylpyrazin-2-amine;
(k) 6-(((1S,4S)-5-(3-chloro-2-fluorobenzoyl)-2,5-diazabicyclo(2.2.1)hept-2-yl)méthyl)-N-1H-pyrazol-3-ylpyrazin-2-amine;
(l) 6-((4-(3-fluoro-3-(trifluorométhyl)benzoyl)pipérazin-1-yl)méthyl)-N-1H-pyrazol-3-ylpyrazin-2-amine;
(m) 6-((4-(3-chloro-2-fluorobenzoyl)pipérazin-1-yl)méthyl)-N-(5-méthyl-1H-pyrazol-3-yl)pyrazin-2-amine;
(n) 6-((4-(3-chloro-2-fluorobenzoyl)pipérazin-1-yl)méthyl)-N-thiazol-2-ylpyrazin-2-amine;
(o) 6-((4-(3-chloro-2-fluorobenzoyl)pipérazin-1-yl)méthyl)-N-(5-chlorothiazol-2-yl)pyridin-2-amine; ou
(p) 6-((4-(2-fluorobenzoyl-3-trifluorométhyl)pipérazin-1-yl)méthyl)-N-(5-méthyl-1H-pyrazol-3-yl)pyrazin-2-amine;
ou un sel pharmaceutiquement acceptable de celles-ci.

3. Composé selon la revendication 2, qui est de la:
6-((4-(3-chloro-2-fluorobenzoyl)pipérazin-1-yl)méthyl)-N-thiazol-2-ylpyridin-2-amine;
ou un sel pharmaceutiquement acceptable de celle-ci.

4. Composé selon la revendication 2, qui est de la:
6-((4-(2-fluoro-3-(trifluorométhyl)benzoyl)pipérazin-1-yl)méthyl)-N-1H-pyrazol-3-ylpyridin-2-amine;
ou un sel pharmaceutiquement acceptable de celle-ci.

5. Composé selon la revendication 2, qui est de la:
6-((4-(2-fluoro-3-(trifluorométhyl)benzoyl)pipérazin-1-yl)méthyl)-N-1H-pyrazol-3-ylpyrazin-2-amine;
ou un sel pharmaceutiquement acceptable de celle-ci.

6. Composé selon la revendication 2, qui est de la:
6-(((1S,4S)-5-(3-chloro-2-fluorobenzoyl)-2,5-diazabicyclo(2.2.1)hept-2-yl)méthyl)-N-1H-pyrazol-3-ylpyrazin-2-amine;
ou un sel pharmaceutiquement acceptable de celle-ci.

7. Composition pharmaceutique comprenant un composé selon l'une quelconque des revendications précédentes ou un sel pharmaceutiquement acceptable de celui-ci ensemble avec un véhicule ou un diluant pharmaceutiquement acceptable.

8. Composé selon l'une quelconque des revendications 1 à 6 ou un sel pharmaceutiquement acceptable de celui-ci, à utiliser en thérapie.

9. Combinaison d'un composé selon l'une quelconque des revendications 1 à 6 ou d'un sel pharmaceutiquement acceptable de celui-ci et d'au moins un autre agent antitumoral pour administration simultanée, séparée ou séquentielle.

10. Combinaison d'une:
* préparation comprenant, ensemble avec un véhicule ou un diluant pharmaceutiquement acceptable, un composé selon l'une quelconque des revendications 1 à 6; et
* préparation comprenant, ensemble avec un véhicule ou un diluant pharmaceutiquement acceptable, un agent antitumoral sélectionné parmi le groupe consistant en des agents alkylants antitumoraux, des antimétabolites antitumoraux, des antibiotiques antitumoraux, des agents antitumoraux dérivés de plantes, des composés antitumoraux de complexes de platine, des dérivés antitumoraux de campthotécine, des inhibiteurs antitumoraux de tyrosine kinase, des anticorps monoclonaux, des interférons, des modificateurs de réponse biologique et d'autres agents antitumoraux, ou un sel pharmaceutiquement acceptable de celui-ci, où:
les agents alkylants antitumoraux sont le N-oxyde de la moutarde azotée, le cyclophosphamide, l'ifosfamide, le melphalan, le busulfan, le mitobronitol, le carboquone, le thiotépa, la ranimustine, la nimustine, le témozolomide et la carmustine;
les antimétabolites antitumoraux sont le méthotrexate, le 6-mercaptopurine riboside, la mercaptopurine, le 5-fluorouracile, le tégafur, la doxifluridine, le carmofur, la cytarabine, l'ocfosfate de cytarabine, l'énocitabine, S-1, la gemcitabine, la fludarabine et le pemetrexed disodique;
les antibiotiques antitumoraux sont l'actinomycine D, la doxorubicine, la daunorubicine, la néocarzinostatine, la bléomycine, la péplomycine, la mitomycine C, l'aclarubicine, la pirarubicine, l'épirubicine, la zinostatine stimalamère, l'idarubicine, le sirolimus et la valrubicine;
les agents antitumoraux dérivés de plantes sont la vincristine, la vinblastine, la vindesine, l'étoposide, le subuzoxane, le docétaxel, le paclitaxel et la vinorelbine;
les composés antitumoraux de complexes de platine sont le cisplatine, le carboplatine, le nédaplatine et l'oxaliplatine;
les dérivés antitumoraux de campthotécine sont l'irinotécan, le topotécan et la campthotécine;
les inhibiteurs antitumoraux de tyrosine kinase sont le gefitinib, l'imatinib et l'erlotinib;
les anticorps monoclonaux sont le cétuximab, le bévacizumab, le rituximab, le bévacizumab, l'alemtuzumab et le trastuzumab;
les intéférons sont l'interféron α, l'inteféron α-2a, l'interféron α-2b, l'interféron β, l'interféron γ-1a et l'interféron γ-n1;
les modificateurs de réponse biologique sont le Krestin, le lentinan, le sizofiran, le picibanil ou l'ubénimex; et
les autres agents antitumoraux sont le mitoxantrone, la L-asparaginase, la procarbazine, la dacarbazine, l'hydroxy-carbamide, la pentostatine, la trétinoïne, l'aléfacept, la darbépoétine alfa, l'anastrozole, l'exémestane, le bicalutamide, la leuproréline, le flutamide, le fulvestrant, le pégaptanib octasodium, le dénileukine diftitox, l'aldesleukine, la thyrotropine alfa, le trioxyde d'arsenic, le bortézomib, la capécitabine et la goséréline, pour administration simultanée, séparée ou séquentielle.

11. Combinaison selon la revendication 10, dans laquelle l'agent antitumoral est le paclitaxel ou le docétaxel.

12. Utilisation d'un composé selon l'une quelconque des revendications 1 à 6 ou d'un sel pharmaceutiquement acceptable de celui-ci, dans la fabrication d'un médicament pour le traitement ou la prévention du cancer.

13. Utilisation d'une combinaison selon l'une quelconque des revendications 9 à 11, dans la fabrication d'un médicament pour le traitement ou la prévention du cancer.

14. Composé selon l'une quelconque des revendications 1 à 6 ou un sel pharmaceutiquement acceptable de celui-ci, à utiliser dans une méthode de traitement ou de prévention du cancer.


### SEQUENCE LISTING

<110> Banyu Pharmaceutical Co., Ltd.
   Ohkubo, Mitsuru
   Kato, Tetsuya
   Kawanishi, Nobuhiko
   Mita, Takashi
   Shimomura, Toshiyasu
<120> NOVEL AMINOPYRIDINE DERIVATIVES HAVING
   AURORA A SELECTIVE INHIBITORY ACTION
<130> BY0045YS
<150> JP2004-315152
   <151> 2004-10-29
<150> JP2005-161156
   <151> 2005-06-01
<160> 1
<170> FastSEQ for Windows Version 4.0
<210> 1
   <211> 7
   <212> PRT
   <213> Artificial Sequence
<220>
   <223> Completely Synthetic Amino Acid Sequence
<400> 1
   Leu Arg Arg Ala Ser Lys Gly
    1               5